# EUROPEAN PATENT APPLICATION

(11) **EP 3 750 892 A1**
(43) Date of publication of application: **16.12.2020**
(21) Application number: 19305763.5
(22) Date of filing: 14.06.2019
(51) Int. Cl.: C07D 491/048, A61P 35/00, A61K 31/4355

(54) **NOVEL 5-CYCLOPROPYL-FURO[3,4-C]PYRIDINE-3,4(1H,5H)-DIONE 1,1' SUBSTITUTED DERIVATIVES AND THEIR USES**

(71) Applicant: Yerevan State University, 0025 Yerevan (AM); Centre National de la Recherche Scientifique, 75016 Paris (FR); UNIVERSITE PARIS DESCARTES, 75006 Paris (FR)
(72) Inventor: MELIKYAN, Gagik, 0010 Erevan (AM); HAYEK, Simon, 94230 Cachan (FR); PIETRANCOSTA, Nicolas, 78180 Montigny le Bretonneux (FR); HERBEUVAL, Jean-Philippe, 75015 PARIS (FR); ALVES DE SOUZA, Rodolphe, 94200 Ivry sur Seine (FR); VIDALAIN, Pierre-Olivier, 92800 Puteaux (FR)
(74) Representative: A.P.I. Conseil

(57) **Abstract**

Invention provides novel 5-cyclopropyl-furo[3,4-c]pyridine-3,4(1H,5H)-dione 1,1' substituted derivatives of general formula (I) as defined herein. More particularly invention provides methods of preparing said compounds, pharmaceutical compositions and combined therapies comprising said compounds, as well as their use as a medicament (alone or in combination with others active agent) in particular for treating or preventing hyperproliferative disorders.

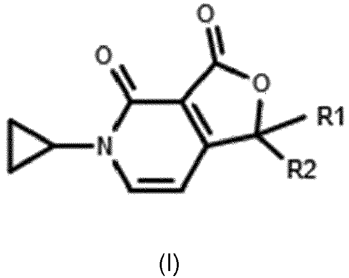

## Description

DNA and RNA synthesis require large amounts of purine and pyrimidine nucleosides as precursors. Further, certain nucleotides like UDP and UTP are implicated in numerous metabolic or signalling pathways. Although cells can recycle or scavenge nucleosides from extracellular medium, this is not sufficient for quickly dividing cells, due to their high rate of DNA and RNA synthesis to sustain cell division. These cells are supposed to be highly dependent on *de novo* nucleoside biosynthesis.

Dihydroorotate dehydrogenase (DHODH) is the rate limiting enzyme of the *de novo* pyrimidine biosynthesis pathway catalysing the fourth step of oxidizing the dihydroorotate to orotate which, downstream, is converted into uridine monophosphate (UMP) that serves as a precursor for cytidine and thymidine biosynthesis. Therefore, inhibition of DHODH is considered as clinically relevant for numerous conditions like cancer, immunological, parasitic and viral diseases (Madak *et al.* 2019).

In the 90s, a potent DHODH inhibitor called brequinar passed pre-clinical studies, and was evaluated for its antitumor properties in multiple phase-I and II trials. Some unacceptable adverse effects and the lack of efficacy found for different types of solid tumors, probably due to improper dosing regimens in regard with its mode of action, led to stop its development. The only two DHODH inhibitors on market today are teriflunomide and leflunomide (its prodrug) for the treatment of rheumatoid arthritis and multiple sclerosis (Madak *et al.* 2019). These drugs were shown to interfere with the proliferation of immune cells by inhibiting both pyrimidine biosynthesis and various tyrosine kinases, which altogether accounts for their immunosuppressive properties. Nevertheless, they are known as weak DHODH inhibitors.

Despite the failure of brequinar, the quest for efficient DHODH inhibitors knows a renewed interest. Acute Myeloid Leukemia and other hematologic malignancies are indications of particular interest because of cytotoxic and prodifferenciation effect of DHODH inhibitors (Sykes, 2018). Today at least 12 ongoing phase I or II clinical trials related to the use of DHODH inhibitors in the treatment of cancer, inflammatory or immunological diseases are listed in clinical trial website (https://clinicaltrials.gov/).

WO 2018/077923 and WO 2018/07792 disclose DHODH inhibitors useful for treating hyperproliferative disorders.

WO2017/117372 discloses DHODH inhibitors useful in cancer bearing mutations in IDH1 or IDH 2 genes.

US 2012/0035175 relates to salts of inhibitors of DHODH as well as to their use as anti-inflammatory, immunomodulatory and anti-proliferating agents.

WO 2008/077639 discloses amino nicotinic and isonicotinic acid derivatives as DHODH inhibitors.

In this context, the identification of novel DHODH inhibitors with original chemical scaffolds to overcome the limitations of existing molecules and to provide further drug candidates is of particular interest.

### SUMMARY

Invention thus relates to novel DHODH inhibitors. Through a comprehensive analysis of DHODH structure and its interaction with molecules inventors have been able to develop a novel method of in silico screening which allow them to identify DHODH inhibitors exhibiting a totally new scaffold. These compounds are novel 5-cyclopropyl- [3,4-c]pyridine-3,4(1H,5H)-dione 1,1' substituted derivatives, which as described in the experimental section share common properties with known DHDOH inhibitors and then constitute valuable candidates as novel DHODH inhibitors.

The present invention relates thus a compound of general formula I, its salts, solvates and / or stereoisomers, 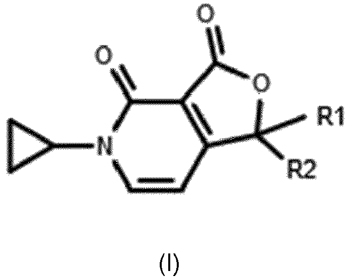
- in which R1 and R2 each represent, independently from each other a group selected from
   ∘ a (C1-C8)alkyl or heteroalkyl,
   ∘ a (C3-C6) cycloalkyl or a 3- to 6- membered heterocycloalkyl,
   ∘ a (C3-C6) cycloalkenyl or 3- to 6- membered heterocycloalkenyl,
   ∘ a phenyl, a tolyl, or a phenyl-,cyclohexyl-or heterocycle-substituted phenyl,
   ∘ a cycloalkyl-(C1-C8)alkyl or a heterocycloalkyl-(C1-C8)alkyl,
   ∘ a cycloalkenyl-(C1-C8)alkyl or a heterocycloalkenyl-(C1-C8)alkyl, or
   ∘ an aryl-(C1-C8)alkyl, or a heteroaryl-(C1-C8)alkyl,
   with the proviso that at least one of R1 or R2 is a group selected from :
   ∘ a (C3-C6) cycloalkyl or a 3- to 6- membered heterocycloalkyl,
   ∘ a (C3-C6) cycloalkenyl or 3- to 6- membered heterocycloalkenyl,
   ∘ a phenyl, a tolyl, or a phenyl-,cyclohexyl-or heterocycle-substituted phenyl,
   ∘ a cycloalkyl-(C1-C8)alkyl or a heterocycloalkyl-(C1-C8)alkyl,
   ∘ a cycloalkenyl-(C1-C8)alkyl or a heterocycloalkenyl-(C1-C8)alkyl, or
   ∘ an aryl (C1-C8)alkyl, or a heteroaryl (C1-C8)alkyl,
   or
- R1 and R2 form together a bond or a (C3-C8) cycloalkyl a 3- to 8- membered heterocycloalkyl, a (C3-C8) cycloalkenyl, or a 3- to 8- membered heterocycloalkenyl.

In particular, the invention relates to a compound of formula I, its salts, solvates and / or stereoisomers in which:
- R1 is a (C1-C8) alkyl, and
- R2 is a group selected from :
   - a (C3-C6) cycloalkyl or a 3- to 6- membered heterocycloalkyl,
   - a (C3-C6) cycloalkenyl or 3- to 6- membered heterocycloalkenyl,
   - a cycloalkyl-(C1-C8)alkyl or a heterocycloalkyl-(C1-C8)alkyl,
   - a cycloalkenyl-(C1-C8)alkyl or a heterocycloalkenyl-(C1-C8)alkyl, or
   - an aryl (C1-C8)alkyl, or a heteroaryl (C1-C8)alkyl.

More particularly, the invention relates to a compound of formula I, its salts, solvates and / or stereoisomers wherein :
- R1 is a (C1-C8) alkyl, and
- R2 is a group selected from a phenyl, naphtyl-(C1-C8) alkyl, indolyl-(C1-C8) alkyl, pyrrolyl-(C1-C8) alkyl, furanyl-(C1-C8) alkyl, aryl-(C1-C8) alkyl, heteroaryl-(C1-C8) alkyl, cyclopentadienyl-(C1-C8) alkyl, heterocyclopentandienyl-(C1-C8) alkyl, said group being optionally substituted by one or more group selected independently from (C1-C6) alkyl, halogen atom or N, O, S, methoxy or nitro groups.

In another embodiment, particular compounds according to the invention are compounds of formula I, wherein R1 and R2 form together a (C3-C8) cycloalkyl, optionally substituted by one or more group selected independently from (C1-C6) alkyl, halogen atom, amine, hydroxy, thiol, methoxy or nitro group, its salts, solvates and / or stereoisomers.

Even more particularly, the invention relates to particular compounds of formula I as listed in table I.

Because of their advantageous biological activities as described herein, the invention also relates to a compound according to the invention for its use as a medicament. Accordingly, invention relates also to a pharmaceutical composition comprising at least one of said compounds.

Compounds of the invention are effective DHDOH inhibitors, and as such, inhibit pyrimidine *de novo* biosynthesis; therefore, the invention also relates to their use in treating or preventing a disease responsive to pyrimidine *de novo* biosynthesis inhibitors, in a particular, a proliferative disorder.

In particular, the invention also relates to a compound according to formula I for its use in reducing proliferation of a cell in a subject in need thereof.

More particularly, the invention relates to a compounds of formula I for its use in treating a disease selected from :
- cancer, preferably selected from small and large intestine cancer, lung cancer, e.g. *RAS*/*LKB1* double-mutant lung cancer, hepatocellular carcinoma (HCC), haematopoietic or lymphoid cancer, Acute Myeloid Leukemia (AML), ovary cancer, PTEN deficient or triple negative breast cancer, KRAS mutant pancreatic cancer, malignant melanoma, e.g. BRAF^{V600E}-mutant melanoma,
- autoimmune diseases or immune and inflammatory diseases, e.g. psoriasis, multiple sclerosis, relapsing remitting multiple sclerosis, rheumatoid arthritis, ulcerative colitis or Crohn's disease, lupus erythematosus, graft versus host disease, graft/transplant rejection. or
- viral infections, e.g. infection to influenza virus, dengue virus, chikungunya virus, human cytomegalovirus, human respiratory syncytial virus, measles virus, coxsackieviruses, rotavirus, Ebola virus, hepatitis B, C or E viruses.

Further, compound of formula I are for instance found to block cell proliferation and / or amplify response to interferon type I or II. Thus, they provide valuable complementary treatment to be combined with at least one chemotherapeutic agent and/or immune checkpoint inhibitor and/or Interferon type I or II agonist, in order to gain in efficacy and/or lowering doses and respective side effects. Accordingly, a particular object of the invention relates to a compound of formula I for its use in treating cancer, said use further comprising the combined or separated, simultaneous or sequential, use of at least one chemotherapeutic agent and/or immune checkpoint inhibitor and/or Interferon type I or II agonist. More particularly, another object of the invention relates to a compound according to the invention for its use in therapies and uses as exposed above further comprising the combined or separated, simultaneous or sequential use of at least one compound selected from cyclophosphamide, mechlorethamine, chlorambucil, melphalan, dacarbazine, nitrosoureas, temozolomide, daunorubicin, doxorubicin, epirubicin, idarubicin, mitoxantrone, valrubicin, paclitaxel, docetaxel, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, mercaptopurine, methotrexate, tioguanine, bleomycin, actinomycin, carboplatin, cisplatin, oxaliplatin, tretinoin, alitretinoin, bexarotene, vinblastine, vincristine, vindesine, vinorelbine, nivolumab, pembrolizumab, atezolizumab, avelumab, durvalumab, cemiplimab, pidilizumab, AMP-224, AMP-514, PDR001, BMS-936559, CK-301, imiquimod, dactolisib, gardiquimod, esiquimod or sumanirole or other Imidazoquinoline derivative, bevacizumab, thalidomide, lenalidomide, sorafenib, sunitinib, temsirolimus, axitinib, pazopanib, cabozantinib, everolimus, ramucirumab, regorafenib, vandetanib, aflibercept or ziv-aflibercept interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a, salts or prodrugs or derivatives or sustained release formulations thereof or a combination thereof.

Also, pyrimidine biosynthesis pathway is found at the crossroad with the innate immunity and DNA damages pathways. Accordingly, compounds of the invention provide valuable tools to study those mechanisms. The invention thus also relates to the use of a compound according to formula 1 as dihydroorotate dehydrogenase (DHODH) inhibitor in an *in vitro* assay.

Further advantages and features of the invention are exposed in the following description hereby provided in an illustrative and a non-limitative way.

### FIGURE LEGENDS

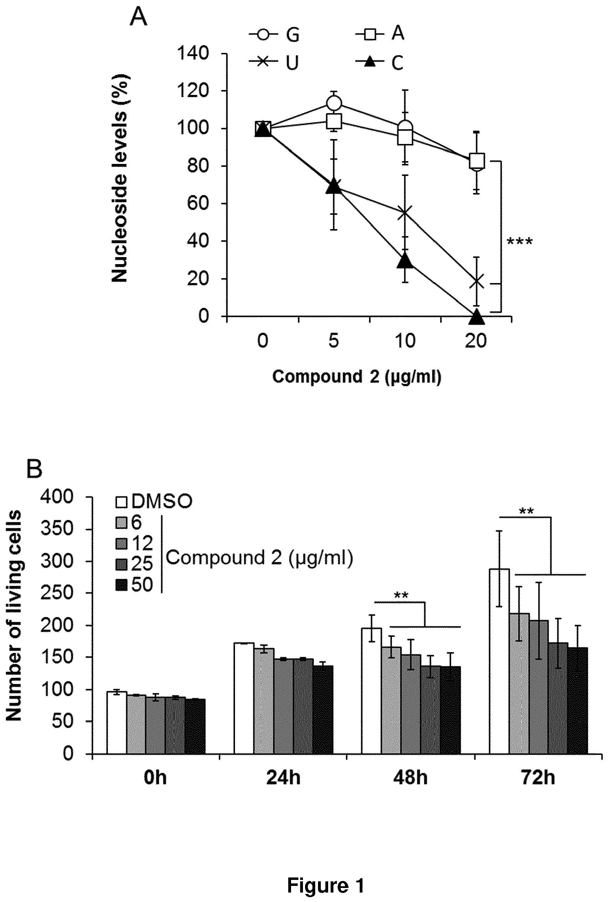

**Figure 1****: Compounds of the invention are inhibiting *de novo* pyrimidine biosynthesis. (A)** Incubation with compounds of the invention result in a drop of pyrimidine cellular concentration. Cells were treated with DMSO or compound 2 (0; 5; 10 or 20 µg/mL). After 24 h of culture, cells were harvested and intracellular levels of purine (G/A) and pyrimidine (C/U) were determined by HPLC-coupled spectrophotometry. Results were normalized using DMSO-treated cells as reference. Data represent means +/- SD of three independent experiments. ***, p<0.001 as calculated by two-way ANOVA. **(B)** Long term incubation with compounds of the invention prevent cell proliferation when compared with non-treated cell cultures. Cells were treated with DMSO or compound 2 at different concentrations, and the number of viable cells was determined after 24, 48 and 72 h of culture. Data represent means +/-SD of two independent experiments. **, p<0.01 as calculated by one-way analysis of variance (ANOVA) with Bonferroni's post hoc test.

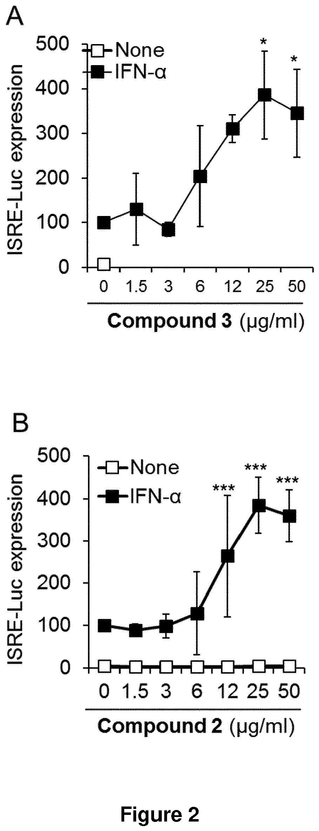

**Figure 2****: Cellular response to IFN-α is amplified by compounds of the invention.** HEK-293 cells with the ISRE-Luc reporter gene (STING 37 cell line) were treated with different concentration of compound 2 (A), compound 3 (B) (black squares) or DMSO alone in the absence or presence of IFN-α (150 IU/mL, open squares). Data represent means +/- SD of three independent experiments. *, p<0.05; ***, p<0.001 as calculated by one-way analysis of variance (ANOVA) with Bonferroni's post hoc test.

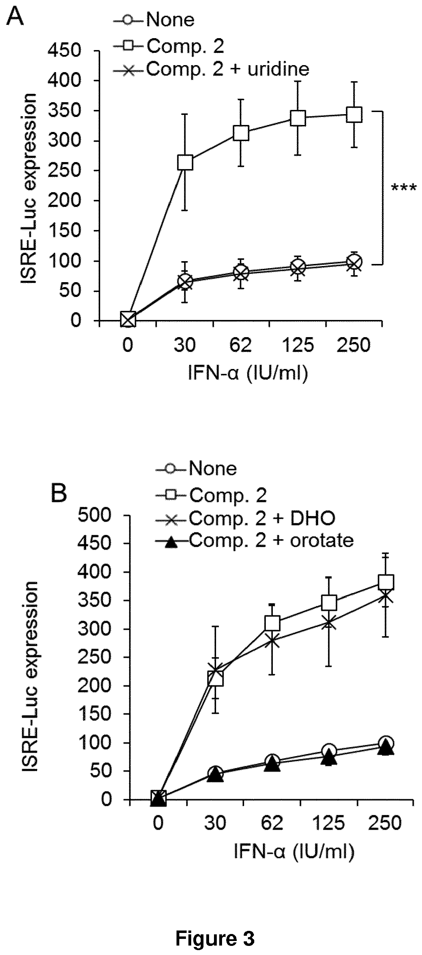

**Figure 3****: Amplification of response to IFN-α by compounds of the invention is reversed by orotate but not DHO.** HEK-293 cells with the ISRE-Luc reporter gene (STING 37 cell line) were treated with increasing concentrations of IFN-α with DMSO or compound 2 (80 µM), and cotreated or not with uridine (125 µM, (A)), DHO (3 mM, (B)), or Orotate (3 mM, (B)). Results were normalized using cells treated with DMSO + IFN-α at 250 IU/mL as reference. Data represent means +/- SD of three independent experiments. ***, p<0.001 as calculated by two-way ANOVA.

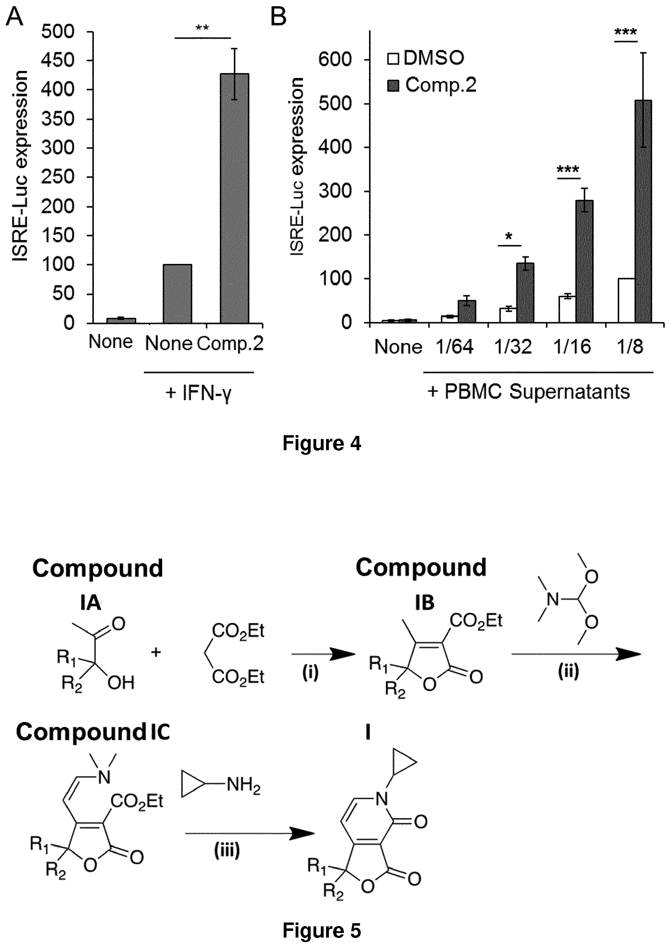

- **Figure 4** : **Amplification of response to IFN type II by compounds of the invention.** (A) HEK-293 cells with the ISRE-Luc reporter gene (STING 37 cell line) were treated with DMSO or compound 2 (80 µM), in the absence or presence of recombinant IFN-γ. Results were normalized using cells treated with DMSO + IFN-γ as reference. **, p<0.01 as calculated by two-tailed t-test. **(B)** Same experiment as in (A), but cells were stimulated with different dilutions ranging from 1/8 to 1/64 of conditioned supernatants from activated PBMCs. Results were normalized using cells treated with 1/8 diluted supernatants as reference. Data represent means +/- SD of three independent experiments. *, p<0.05; *** p<0.001 determined by two-way ANOVA with Bonferroni's post hoc test.
- **Figure 5****: Scheme of synthesis of 5-cyclopropyl-[3,4-c]pyridine-3,4(1H,5H)-dione 1,1' substituted derivatives according to the invention.**

### DETAILED DESCRIPTION AND ADDITIONAL EMBODIMENTS

Using a proprietary *in silico* screening, inventors have been able to identify new compounds which are able to inhibit DHODH enzyme. As shown in the experimental section, these compounds are novel 5-cyclopropyl- [3,4-c]pyridine-3,4(1H,5H)-dione 1,1' substituted derivatives which, beside complying with the rules of screening developed by the inventors, are found to constitute a group of novel DHODH inhibitors capable of inhibiting cell proliferation and amplifying type I and/or type II interferon response.

### Definitions

The term "(C1-C8)alkyl" as used in the present invention refers to a saturated or unsaturated, linear or branched hydrocarbon chain comprising from 1 to 8 carbon atoms, including, but not limited to, methyl, ethyl, n-propyl, isopropyl, n-butyl, isobutyl, sec-butyl, tert-butyl, n-pentyl, n-hexyl, n-heptyl and n-octyl and the like, when related to saturated or branched saturated hydrocarbon chain, or including, but not limited to, ethenyl (e.g. vinyl), propenyl (e.g. allyl), butenyl, pentenyl, hexenyl and the like, when related to unsaturated hydrocarbon chain or branched unsaturated hydrocarbon chain.

The term "hetero alkyl" as used in the present invention refers to an alkyl chain as defined above including heteroatoms such as, but not limited to, N, O, S.

The term "cycloalkyl" as used in the present invention refers to a saturated hydrocarbon ring comprising from 3 to 8 (in other words, (C3-C8) cycloalkyl), advantageously from 3 to 6, carbon atoms including, but not limited to, cyclohexyl, cyclopentyl, cyclopropyl, cyclobutyl, cycloheptyl, cyclooctyl and the like.

The term "cycloalkenyl" as used in the present invention is related to a saturated hydrocarbon ring comprising from 3 to 8 (in other words, (C3-C8) cycloalkyl), advantageously from 3 to 6, carbon atoms including, but not limited to, cyclobutenyl, cyclopentenyl, cyclopentandienyl cyclohexenyl, cyclohexadienyl and the like. Accordingly, in an embodiment said cycloalkenyl can comprise one or two double bonds.

The terms "heterocycloalkyl" or "heterocycloalkenyl" correspond to a "cycloalkyl" or a "cycloalkenyl" residue respectively as described above including heteroatoms (e.g. N, O, S) such as, but not limited to pyrrolyl, furanyl, thiophenyl, thiiranyl, thietanyl, oxiranyl, oxetanyl, furanyl, aziridinyl, azetidinyl, pyrrolidinyl, piperidinyl and the like.

The term "aryl", as used in the present invention, refers to an aromatic hydrocarbon group comprising preferably 5 to 10 carbon atoms and comprising one or more fused rings, such as, for example, a cyclopentandienyl, a phenyl, or naphthyl. Advantageously, it will be a phenyl group.

The term "heteroaryl" as used in the present invention refers to an aromatic heterocycle as defined above. It can be more particularly an aromatic monocyclic, or bicyclic, each cycle comprising 5 or 6 members, such as a pyrrole, a furan, a thiophene, a thiazole, an isothiazole, an oxazole, an isoxazole, an imidazole, a pyrazole, a triazole, a pyridine, a pyrimidine, an indole, a benzofuran, a benzothiophene, a benzothiazole, a benzoxazole, a benzimidazole, an indazole, a benzotriazole, a quinoline, an isoquinoline, a quinazoline, a quinoxaline and the like. Accordingly, the term heterophenyl, as used in the present invention refers to a phenyl group, said phenyl group comprising at least one hetero atom (e.g. N, O or S) instead of at least one carbon, such as, but not limited to a pyridine or a pyrimidine.

According the present invention, each of these groups within compounds of the invention is optionally substituted by one or more group selected independently from (C1-C6) alkyl, halogen atom, amine, hydroxy, thiol, methoxy or nitro groups or the like.

The term "C1-C8" as used in the present invention to define (C1-C8) alkyl, (C1-C8) heteroalkyl, (C1-C8) cycloalkyl or (C1-C8) heterocycloalkyl, (C1-C8) cycloalkenyl or (C1-C8) heterocycloalkenyl means respectively an alkyl, heteroalkyl, cycloalkyl, heterocycloalkyl, cycloalkenyl or heterocycloalkenyl group having a finite number of carbon or members, *i.e.* 1, 2, 3, 4, 5, 6, 7 or 8 carbon atoms. In the same way, the term "C1-C6" as used herein to define a "C1-C6" alkyl means an alkyl group having a finite number of carbon or members, *i.e.* 1, 2, 3, 4, 5 or 6 carbon atoms. As used herein, the term "C3-C8" used e.g. in the context of the definition of cycloalkyl, hetero cycloalkyl, cycloalkenyl, hetero cycloalkenyl means a cycloalkyl, hetero cycloalkyl, cycloalkenyl, hetero cycloalkenyl group having 3 to 8 atoms of carbon atoms or members, i.e. 3, 4, 5, 6, 7 or 8 carbon atoms. Also, as used herein, the term C3-C6 used e.g. in the context of the definition of cycloalkyl, hetero cycloalkyl, cycloalkenyl or hetero cycloalkenyl means a cycloalkyl, hetero cycloalkyl, cycloalkenyl or hetero cycloalkenyl group having 3 to 6 atoms of carbon atoms or members, i.e. 3, 4, 5, 6 carbon atoms or members.

The terms "cycloalkyl-(C1-C8)alkyl" or "cycloakenyl-(C1-C8)alkyl" as used in the present invention refer to any cycloalkyl or cycloalkenyl group as defined above, which is bound to the molecule by means of a (C1-C8)-alkyl group as defined above.

The term "heterocycloalkyl-(C1-C8)alkyl" or "heterocycloalkenyl-(C1-C8)alkyl" as used in the present invention refers to a heterocycloalkyl or heterocycloalkenyl group as defined above, which is bound to the molecule by means of a (C1-C8)-alkyl group as defined above.

The term "aryl-(C1-C8)-alkyl" as used in the present invention refers to any aryl group as defined above, which is bound to the molecule by means of a (C1-C8)-alkyl group as defined above. In particular, it can be a benzyl, methoxybenzyl, nitrobenzyl or a fluorobenzyl group.

The term "heteroaryl-(C1-C8)alkyl" as used in the present invention refers to a heteroaryl group as defined above, which is bound to the molecule by means of a (C1-C8)-alkyl group as defined above.

The term "halogen" as used in the present invention refers to an atom of fluorine, bromine, chlorine or iodine. Preferably, this is an atom of fluorine.

As used herein, "treatment" includes the therapy, prevention, prophylaxis, retardation or reduction of symptoms provoked by or of the causes of a disease. When related to a disease responsive to pyrimidine *de novo* biosynthesis inhibitors, said disease comprises disease or disorders in which DHODH inhibition plays a role either through an inhibition of cell proliferation and/or through the amplification of response to type I or II interferons, *i.e.* hyperproliferative disorders, autoimmune diseases, immune and inflammatory diseases, viral diseases or cancer.

According to the invention, the term "treat" also includes stopping, stabilizing or slowing down the progression of a disease, or lessening the severity of its symptoms.

"Hyperproliferative disorders" or "proliferative disorders" are used herein interchangeably. These disorders are characterized by an uncontrolled or an abnormally high rate of proliferation of cells. For example, said diseases can be cancer, comprising, but not limited to leukemias, lymphomas, myelomas, skin disorders like, but not limited to, psoriasis, or even hyperplasias.

"Autoimmune diseases or immune and/or inflammatory diseases" which may be prevented or treated with compounds of the invention comprise, but are not limited to, psoriasis, multiple sclerosis, relapsing remitting multiple sclerosis, rheumatoid arthritis, ulcerative colitis or Crohn's disease, lupus erythematosus, graft versus host disease, graft/transplant rejection.

"Viral infection" which may be prevented or treated with compounds of the invention comprise but are not limited to infection to influenza virus, dengue virus, chikungunya virus, human cytomegalovirus, human respiratory syncytial virus, measles virus, coxsackieviruses, rotavirus, Ebola virus, hepatitis B, C or E viruses, and so on.

"Cancer" as used herein refers to a group of diseases involving abnormal and uncontrolled cell proliferation within an organ or the body, with a potential to invade or spread to other parts of the body. Basically, most of the cancers can be prevented or treated with compounds of the invention; more particularly, their progression can be stopped, stabilized or slowed, or associated symptoms can be lessened using compounds of the invention. In a particular embodiment, "Cancer" designates any cancer known to be treatable either partially or completely by the administration a DHODH inhibitor e.g. comprises, but is not limited to, small and large intestine cancer, lung cancer, e.g. *RAS*/*LKB1* double-mutant lung cancer, hepatocellular carcinoma (HCC), haematopoietic or lymphoid cancer, myeloid malignancies as myelodysplastic syndromes, myeloproliferative neoplasms, chronic myelomonocytic leukemia or Acute Myeloid Leukemia (AML), ovary cancer, PTEN deficient or triple negative breast cancer, KRAS mutant pancreatic cancer or malignant melanoma, e.g. BRAF^{V600E}-mutant melanoma.

As used herein, the term "compound" or "drug" designates the chemical compound or drug as named in the application, as well as any pharmaceutically acceptable salt, hydrate, ester, ether, stereoisomer, racemate, conjugate, pro-drug thereof, of any purity.

The terms "Combination" or "combinatorial treating/therapy" as used herein designate a treatment of a disease or disorder wherein at least two or more drugs and/or compounds are co-administered to a subject to cause a biological effect, at least one of the said drugs and/or compounds being a compound of the invention. In a combined therapy according to this invention, the at least two drugs and/or compounds may be administered at the same time, together or separately, or sequentially. Also, the at least two drugs and/or compounds may be administered through different routes and protocols. As a result, although they may be formulated together, the drugs and/or compounds of a combination may also be formulated separately.

### Compounds of the invention

In a particular embodiment, R1 is a (C1-C8) alkyl, and R2 is a group selected from phenyl, phenyl-ethyl, phenyl-propyl, cyclopentadienyl-ethyl, cyclopentadienyl-propyl, heterocyclopentandienyl-ethyl, heterocyclopentandienyl-propyl, napthyl-ethyl, napthyl-propyl, indolyl-ethyl, indolyl-propyl, pyrrolyl-ethyl, pyrrolyl-propyl, furyl-ethyl, furyl-propyl, - thiophenyl-ethyl, thiophenyl-propyl.

In a another particular embodiment, R1 is a (C1-C8) alkyl, and R2 is a group selected from aryl-(C1-C8) alkyl, 2-, 3- or 4-pyridyl-(C1-C8) alkyl, 1- or 2- thiophenyl-(C1-C8) alkyl, furyl-2-(C1-C8) alkyl, phenyl-(C1-C8) alkyl, fluorophenyl-(C1-C8) alkyl, methoxyphenyl-(C1-C8)alkyl, nitrophenyl-(C1-C8)alkyl, N-methylpyrrol-2- (C1-C8) alkyl et N-methylindol-3-(C1-C8) alkyl.

According to a particular embodiment R1 represents a methyl, and R2 is a group selected from :
- a (C3-C6) cycloalkyl or a 3- to 6- membered heterocycloalkyl,
- a (C3-C6) cycloalkenyl or 3- to 6- membered heterocycloalkenyl,
- a phenyl, a tolyl, or a phenyl-,cyclohexyl-or heterocycle-substituted phenyl,
- a cycloalkyl-(C1-C8)alkyl or a heterocycloalkyl-(C1-C8)alkyl,
- a cycloalkenyl-(C1-C8)alkyl or a heterocycloalkenyl-(C1-C8)alkyl, or
- an aryl-(C1-C8)alkyl, or a heteroaryl-(C1-C8)alkyl.

According to another embodiment, R1 and R2 each represent, independently from each other a group selected from :
- a (C3-C6) cycloalkyl or a 3- to 6- membered heterocycloalkyl,
- a (C3-C6) cycloalkenyl or 3- to 6- membered heterocycloalkenyl,
- a phenyl, a tolyl, or a phenyl-,cyclohexyl-or heterocycle-substituted phenyl,
- a cycloalkyl-(C1-C8)alkyl or a heterocycloalkyl-(C1-C8)alkyl,
- a cycloalkenyl-(C1-C8)alkyl or a heterocycloalkenyl-(C1-C8)alkyl, or
- an aryl-(C1-C8)alkyl, or a heteroaryl-(C1-C8)alkyl.

In a more particular embodiment, when R1 and/or R2 i) represent a (C3-C6) cycloalkenyl, a 3- to 6- membered heterocycloalkenyl, a cycloalkenyl-(C1-C8)alkyl or a heterocycloalkenyl-(C1-C8)alkyl or ii) form together a (C3-C8) cycloalkenyl or a 3- to 8-membered heterocycloalkenyl, then said cycloalkenyl or heterocycloalkenyl, comprise one or two double bond(s).

According to an embodiment, when R1 and/or R2 are selected from a cycloalkyl-(C1-C8)alkyl, a heterocycloalkyl-(C1-C8)alkyl, an aryl (C1-C8)alkyl, or a heteroaryl (C1-C8)alkyl, said -(C1-C8)alkyl member is unsaturated ; even more particularly, it comprises one or two double bond(s).

According to another embodiment, when R1 and/or R2 are selected from a cycloalkyl-(C1-C8)alkyl, a heterocycloalkyl-(C1-C8)alkyl, an aryl (C1-C8)alkyl, or a heteroaryl (C1-C8)alkyl, said -(C1-C8)alkyl part from said R1 and / or R2 group is substituted ; more particularly, said -(C1-C8)alkyl part is substituted by one or more group selected independently from (C1-C6) alkyl, halogen atom, amine, hydroxy, thiol, methoxy or nitro groups or the like ; even more particularly said -(C1-C8)alkyl member is substituted by one or more methyl, preferably one.

In yet another embodiment, when R1 and/or R2 are selected from a cycloalkyl-(C1-C8)alkyl, a heterocycloalkyl-(C1-C8)alkyl, an aryl (C1-C8)alkyl, cycloalkenyl-(C1-C8)alkyl, a heterocycloalkenyl-(C1-C8)alkyl, an aryl (C1-C8)alkyl, or a heteroaryl (C1-C8)alkyl, said cycloalkyl, heterocycloalkyl, cycloalkenyl, heterocycloalkenyl, aryl and/or heteroaryl part from said R1 and / or R2 group is substituted ; more particularly, said cycloalkyl, heterocycloalkyl, cycloalkenyl, heterocycloalkenyl, aryl and/or heteroaryl member part from R1 and/or R2 is substituted by one or more group selected independently from (C1-C6) alkyl, halogen atom, amine, hydroxy, thiol, methoxy or nitro groups or the like. Even more particularly,

In yet another embodiment, when R1 and/or R2 are selected from :
- a (C3-C6) cycloalkyl or a 3- to 6- membered heterocycloalkyl,
- a (C3-C6) cycloalkenyl or 3- to 6- membered heterocycloalkenyl,
- a phenyl, or a phenyl-,cyclohexyl-or heterocycle-substituted phenyl,
- a cycloalkyl-(C1-C8)alkyl or a heterocycloalkyl-(C1-C8)alkyl,
- a cycloalkenyl-(C1-C8)alkyl or a heterocycloalkenyl-(C1-C8)alkyl, or
- an aryl-(C1-C8)alkyl, or a heteroaryl-(C1-C8)alkyl.
said cycloalkyl, cycloalkenyl, phenyl, heterocycloalkyl, cycloalkenyl, aryl or heteroaryl members in said groups are mono or di substituted, preferably with a group selected independently from (C1-C6) alkyl, halogen atom, amine, hydroxy, thiol, methoxy or nitro groups or the like. More particularly said (C3-C6) cycloalkyl, (C3-C6) alkenyl, 3- to 6-membered heterocycloalkyl or 3- to 6- membered heterocycloalkenyl or phenyl are mono or di substituted , preferably with a group selected independently from (C1-C6) alkyl, halogen atom, amine, hydroxy, thiol, methoxy or nitro groups or the like. Even more particularly, said (C3-C6) cycloalkyl, (C3-C6) cycloalkenyl, 3- to 6-membered heterocycloalkyl, 3- to 6-membered heterocycloalkenyl or phenyl are mono or di substituted with a methyl, preferably mono substituted with a methyl. In another particular embodiment said (C3-C6) cycloalkyl or 3- to 6-membered heterocycloalkyl or phenyl are mono or di substituted by a halogen atom, preferably mono substituted by an halogen atom. In another particular embodiment said halogen atom is fluorine.

In a preferred embodiment, R1 and/or R2 are selected from:
- a cyclohexyl or a heterocyclohexyl,
- a phenyl,
- a cyclohexyl-(C1-C8)alkyl or a heterocyclohexyl-(C1-C8)alkyl, or
- an phenyl (C1-C8)alkyl, or a heterophenyl (C1-C8)alkyl, and
said cyclohexyl or heterocyclohexyl, phenyl or heterophenyl are mono or di substituted, preferably at position 3 and/or 4. Even more preferably, the mono-substitution occurs at position 4. In an even more particular embodiment, said cyclohexyl or heterocyclohexyl, phenyl, or heterophenyl are mono or di substituted with a group selected independently from (C1-C6) alkyl, halogen atom, amine, hydroxy, thiol, methoxy or nitro groups or the like.

In a preferred embodiment, R1 and/or R2 are a group selected from indolyl, thiophenyl, furanyl, pyrrolyl, napthyl, indolyl-(C1-C8)alkyl, thiophenyl-(C1-C8)alkyl, furanyl-(C1-C8)alkyl, pyrrolyl-(C1-C8)alkyl, or napthyl-(C1-C8)alkyl groups. In another preferred embodiment said indolyl, thiophenyl, furanyl, pyrrolyl, napthyl or indolyl-, thiophenyl-, furanyl-, pyrrolyl-, or napthalenyl- part in said group are mono or di substituted. In a further particular embodiment, said indolyl, thiophenyl, furanyl, pyrrolyl, napthyl or indolyl-, thiophenyl-, furanyl-, pyrrolyl-, or napthyl- part in said group are mono or di substituted with a group selected independently from (C1-C6) alkyl, halogen atom, amine, hydroxy, thiol, methoxy or nitro groups or the like, even more preferably a methyl.

In yet another embodiment, a compound of the invention is selected from those listed in table 1.

**Table 1**

| **Specific examples of compounds of the invention** | | |
|---|---|---|
| **#** | **Formula** | **Name** |
| 1 | 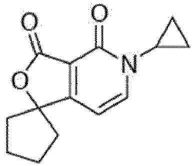 | 5'-cyclopropyl-3'H-spiro[cyclopentane-1,1'-furo[3,4-c]pyridine]-3',4(5'H)-dione |
| 2 | 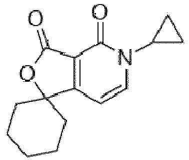 | 5'-cyclopropyl-3'H-spiro[cyclohexane-1,1'-furo[3,4-c]pyridine]-3',4(5'H)-dione |
| 3 | 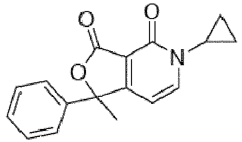 | 5-cyclopropyl-1-methyl-1-phenylfuro[3,4-c]pyridine-3,4(1H,5H)-dione |
| 4 | 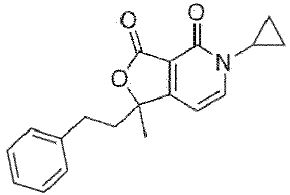 | 5-cyclopropyl-1-methyl-1-phenethylfuro[3,4-c]pyridine-3,4(1H,5H)-dione |
| 5 | 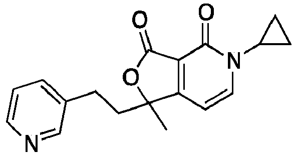 | 5-cyclopropyl-1-methyl-1-(2-(pyridin-3-yl)ethyl)furo[3,4-*c*]pyridine-3,4(1*H*,5*H*)-dione |
| 6 | 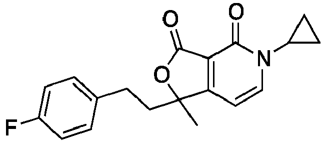 | 5-cyclopropyl-1-(4-fluorophenethyl)-1-methylfuro[3,4-*c*]pyridine-3,4(1*H*,5*H*)-dione |
| 7 | 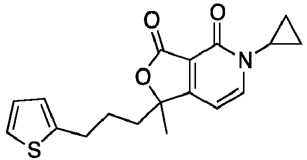 | 5-cyclopropyl-1-methyl-1-(3-(thiophen-2-yl)propyl)furo[3,4-*c*]pyridine-3,4(1*H*,5*H*)-dione |
| 8 | 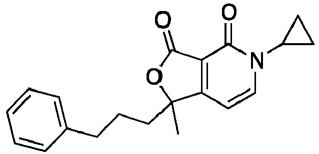 | 5-cyclopropyl-1-methyl-1-(3-phenylpropyl)furo[3,4-*c*]pyridine-3,4(1*H*,5*H*)-dione |
| 9 | 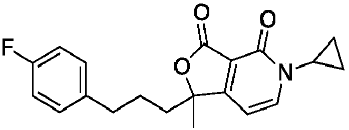 | 5-cyclopropyl-1-(3-(4-fluorophenyl)propyl)-1-methylfuro[3,4-*c*]pyridine-3,4(1*H*,5*H*)-dione |
| 10 | 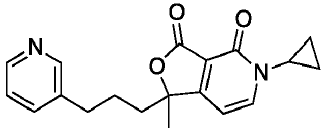 | 5-cyclopropyl-1-methyl-1-(3-(pyridin-3-yl)propyl)furo[3,4-*c*]pyridine-3,4(1*H*,5*H*)-dione |
| 11 | 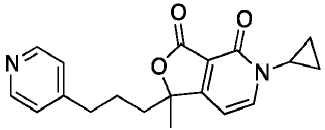 | 5-cyclopropyl-1-methyl-1*-*(3-(pyridin-4-yl)propyl)furo[3,4-*c*]pyridine-3,4(1*H*,5*H*)-dione |
| 12 | 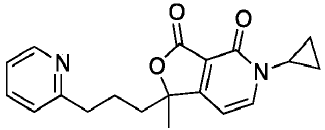 | 5-cyclopropyl-1-methyl-1-(3-(pyridin-2-yl)propyl)furo[3,4-*c*]pyridine-3,4(1*H*,5*H*)-dione |
| 13 | 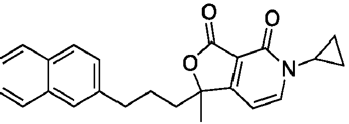 | 5-cyclopropyl-1-methyl-1-(3-(naphthalen-2-yl)propyl)furo[3,4-*c*]pyridine-3,4(1*H*,5*H*)-dione |
| 14 | 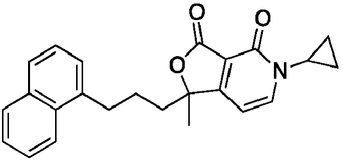 | 5-cyclopropyl-1-methyl-1-(3-(naphthalen-1-yl)propyl)furo[3,4-*c*]pyridine-3,4(1*H*,5*H*)-dione |
| 15 | 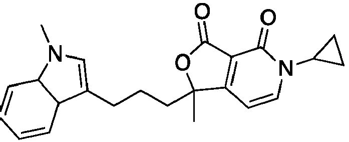 | 5-cyclopropyl-1-methyl-1-(3-(1-methyl-3a,7a-dihydro-1*H*-indol-3-yl)propyl)furo[3,4-*c*]pyridine-3,4(1*H*,5*H*)-dione |
| 16 | 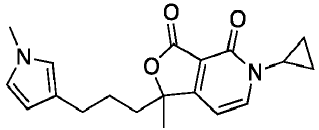 | 5-cyclopropyl-1-methyl-1-(3-(1-methyl-1*H*-pyrrol-3-yl)propyl)furo[3,4-*c*]pyridine-3,4(1*H*,5*H*)-dione |
| 17 | 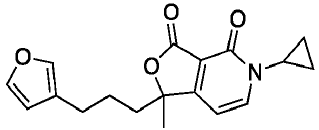 | 5-cyclopropyl-1-(3-(furan-3-yl)propyl)-1-methylfuro[3,4-*c*]pyridine-3,4(1*H*,5*H*)-dione |
| 18 | 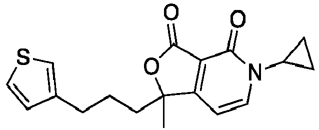 | 5-cyclopropyl-1-methyl-1-(3-(thiophen-3-yl)propyl)furo[3,4-*c*]pyridine-3,4(1*H*,5*H*)-dione |
| 19 | 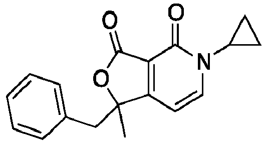 | 1-benzyl-5-cyclopropyl-1-methylfuro[3,4-*c*]pyridine-3,4(1*H*,5*H*)-dione |
| 20 | 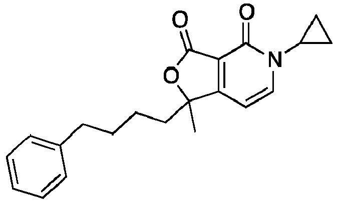 | 5-cyclopropyl-1-methyl-1-(4-phenylbutyl)furo[3,4-*c*]pyridine-3,4(1*H*,5*H*)-dione |
| 21 | 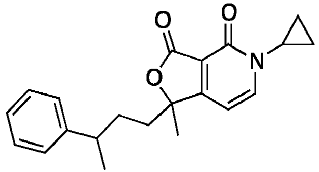 | 5-cyclopropyl-1-methyl-1-(3-phenylbutyl)furo[3,4-*c*]pyridine-3,4(1*H*,5*H*)-dione |
| 22 | 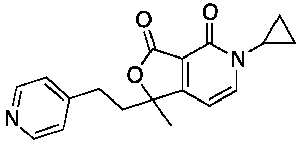 | 5-cyclopropyl-1-methyl-1-(2-(pyridin-4-yl)ethyl)furo[3,4-*c*]pyridine-3,4(1*H*,5*H*)-dione |
| 23 | 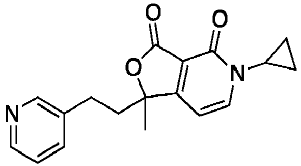 | 5-cyclopropyl-1-methyl-1-(2-(pyridin-3-yl)ethyl)furo[3,4-*c*]pyridine-3,4(1*H*,5*H*)-dione |
| 24 | 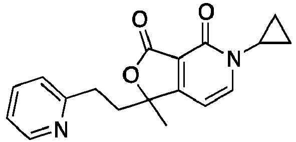 | 5-cyclopropyl-1-methyl-1-(2-(pyridin-2-yl)ethyl)furo[3,4-*c*]pyridine-3,4(1*H*,5*H*)-dione |
| 25 | 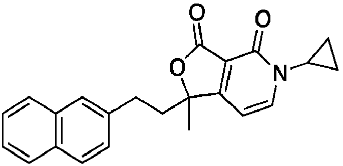 | 5-cyclopropyl-1-methyl-1-(2-(naphthalen-2-yl)ethyl)furo[3,4-*c*]pyridine-3,4(1*H*,5*H*)-dione |
| 26 | 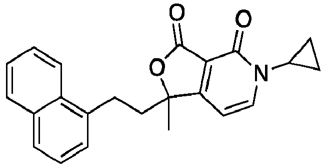 | 5-cyclopropyl-1-methyl-1-(2-(naphthalen-1-yl)ethyl)furo[3,4-*c*]pyridine-3,4(1*H*,5*H*)-dione |
| 27 | 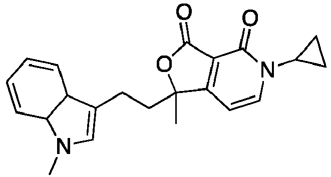 | 5-cyclopropyl-1-methyl-1-(2-(1-methyl-3a,7a-dihydro-1*H*-indol-3-yl)ethyl)furo[3,4-*c*]pyridine-3,4(1*H*,5*H*)-dione |
| 28 | 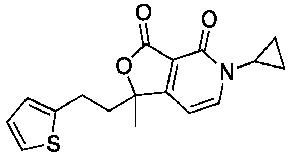 | 5-cyclopropyl-1-methyl-1-(2-(thiophen-2-yl)ethyl)furo[3,4-*c*]pyridine-3,4(1H,5H)-dione |
| 29 | 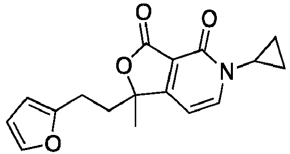 | 5-cyclopropyl-1-(2-(furan-2-yl)ethyl)-1-methylfuro[3,4-*c*]pyridine-3,4(1*H*,5*H*)-dione |
| 30 | 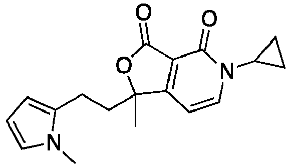 | 5-cyclopropyl-1-methyl-1-(2-(1-methyl-1*H*-pyrrol-2-yl)ethyl)furo[3,4-*c*]pyridine-3,4(1*H*,5*H*)-dione |
| 31 | 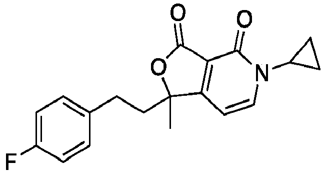 | 5-cyclopropyl-1-(4-fluorophenethyl)-1-methylfuro[3,4-*c*]pyridine-3,4(1*H*,5*H*)-dione |
| 32 | 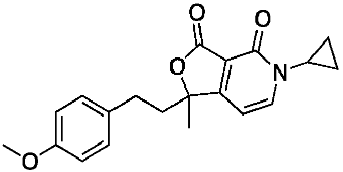 | 5-cyclopropyl-1-(4-methoxyphenethyl)-1-methylfuro[3,4-*c*]pyridine-3,4(1*H*,5*H*)-dione |
| 33 | 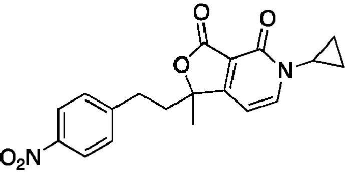 | 5-cyclopropyl-1-methyl-1-(4-nitrophenethyl)furo[3,4-*c*]pyridine-3,4(1*H*,5*H*)-dione |

### Method of synthesis of the compounds of the invention

In another embodiment the invention is related to the process of figure 5 for the production of -cyclopropyl- [3,4-c]pyridine-3,4(1H,5H)-dione 1,1' substituted derivatives of the invention (compounds of formula 1), said process comprising the steps of :

i) Synthesis of compound 1B: refluxing for at least 10 hours, preferably 20 hours a solution of anhydrous ethanol comprising compound 1A and diethylmalonate in a molar ratio of at least 5 preferably 10 (diethyl malonate/compound 1A) in presence of a catalytic amount of sodium ethylate, then reducing the solvent under a reduced pressure and further neutralizing the cooled reaction mixture with diluted hydrochloric acid (6N); proceeding to at least one extraction with diethyl ether, preferably 2 or even more preferably 3, to obtain compound 1B; drying the combined extracts with magnesium sulfate and removing solvent under reduced pressure, the residue being then dried under vacuum before proceeding to step ii),

ii) Synthesis of compound IC: refluxing for at least one hour, preferably 2, even more preferably 3 the product of step i), in an excess of a solution of anhydrous xylene comprising compound of formula 1A and 20% DMF/DMA; then cooling and precipitating the product with diethyl ether. Precipitated crystals are then filtered, washed with diethyl ether and dried under vacuum before proceeding to step iii),

Step iii) Synthesis of compound of formula I: Compound III from step ii) is put to react directly with cyclopropylamine (12 mmol) at least 35°C, preferably 40° for at least 10h, preferably less than 20h, even more preferably for 15 h. The mixture is then cooled to room temperature and precipitated with diethyl ether. Crystals of compounds of formula I are then optionally washed with diethyl ether, filtered and collected.

### Therapeutic methods and uses of the compounds of the invention

As shown in the experimental section, 5-cyclopropyl- [3,4-c]pyridine-3,4(1H,5H)-dione 1,1' substituted derivatives of the invention are actual inhibitors of DHODH. As such they are able to inhibit *de novo* pyrimidine synthesis which results in blocking cellular proliferation, and is linked with an amplification of response to type I interferon. From these properties, compounds of the invention constitute valuable new compounds to be used for treating diseases for which an inhibition of DHDOH is of some interest, e.g., but not limited to, diseases for which blocking or slowing down proliferation of cells or of a particular type of cells (like cancers or autoimmune diseases or immune and/or inflammatory diseases), or for which amplifying the response to interferon response is of some interest (like viral infections or some cancers).

Consequently, in an embodiment, the invention relates to a compound of formula I as described above, or any acceptable salt, hydrate, ester, ether, stereoisomer, racemate, conjugate, pro-drug thereof, of any purity for use as a medicament.

It should understand that compounds of the invention can be used as a medicament in a subject in need thereof, i.e. a subject suffering from, suspected to suffer from or at risk to suffer from a disease for which an inhibition of DHODH might be beneficial.

In a particular embodiment, the invention relates to a compound of formula I as described above, or any acceptable salt, hydrate, ester, ether, stereoisomer, racemate, conjugate, pro-drug thereof, of any purity for use in treating or preventing a disease responsive to pyrimidine *de novo* biosynthesis inhibitors.

In another embodiment, the invention relates to a compound of formula I as described above, or any acceptable salt, hydrate, ester, ether, stereoisomer, racemate, conjugate, pro-drug thereof, of any purity for its use in reducing proliferation of a cell in a subject in need thereof. In a more particular embodiment said subject is suffering from, suspected to suffer from or at risk to suffer from a proliferative disorder. In an even more particular embodiment, said subject is suffering from, suspected to suffer from or at risk to suffer from cancer, said cancer comprising, but being not limited to leukemias, lymphomas, multiple myeolomas, from skin disorders like, but not limited to, psoriasis, or even from hyperplasias.

In an even more particular embodiment the invention relates to a compound of formula I as described above, or any acceptable salt, hydrate, ester, ether, stereoisomer, racemate, conjugate, pro-drug thereof, of any purity for its use in treating an autoimmune disease or immune and/or inflammatory disease, e.g., but not limited to psoriasis, multiple sclerosis, relapsing remitting multiple sclerosis, rheumatoid arthritis, ulcerative colitis or Crohn's disease, lupus erythematosus, graft or transplant rejection, Graft versus Host Disease in a subject in need thereof.

In another particular embodiment the invention relates to a compound of formula I as described above, or any acceptable salt, hydrate, ester, ether, stereoisomer, racemate, conjugate, pro-drug thereof, of any purity for its use in treating a viral infection, e.g., but not limited to infection to influenza virus, dengue virus, chikungunya virus, human cytomegalovirus, human respiratory syncytial virus, measles virus, coxsackieviruses, rotavirus, Ebola virus, hepatitis B,C or E viruses, in a subject in need thereof.

In another particular embodiment the invention relates to a compound of formula I as described above, or any acceptable salt, hydrate, ester, ether, stereoisomer, racemate, conjugate, pro-drug thereof, of any purity for its use in treating cancer. Thought basically all type of cancer inherently implies cell proliferation which should be impaired through the inhibition of *de novo* pyrimidine synthesis, compounds of the invention are of particular use in cancer for which DHODH gene expression is found amplified or protein activity increased, or for which a functional dependency on DHODH activity is documented. Consequently, another object of the invention relates to a compound of formula I as described above, or any acceptable salt, hydrate, ester, ether, stereoisomer, racemate, conjugate, pro-drug thereof, of any purity for its use in treating small and large intestine cancer, lung cancer, e.g; *RAS*/*LKB1* double-mutant lung cancer, hepatocellular carcinoma (HCC), haematopoietic or lymphoid cancer, myeloid malignancies as myelodysplastic syndromes, myeloproliferative neoplasms, chronic myelomonocytic leukemia or Acute Myeloid Leukemia (AML), ovary cancer, PTEN deficient or triple negative breast cancer, KRAS mutant pancreatic cancer or malignant melanoma, e.g. BRAF^{V600E}-mutant melanoma.

In a particular embodiment, this invention relates to a composition comprising at least one compound of formula I as described above, or any acceptable salt, hydrate, ester, ether, stereoisomer, racemate, conjugate, pro-drug thereof, of any purity. In a more particular embodiment, an object of the invention is also said composition for use as a medicament. An even more embodiment this invention relates to said composition for use in any one of the treatments as exposed above.

A composition according to the invention typically comprises one or several pharmaceutically acceptable carriers or excipients. Also, for use in the present invention, compounds of the invention are usually mixed with pharmaceutically acceptable excipients or carriers. Compounds of the invention may be present in an amount of up to 99% by weight of the total weight of the composition. The pharmaceutical composition may be provided in a dosage form that is suitable for the oral, parenteral (e.g., intravenously, intramuscularly), rectal, cutaneous, nasal, vaginal, inhalant, skin (patch), intrathecal or ocular administration route. Thus, the composition may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols. In this regard, in a particular embodiment a composition according to the invention is a pharmaceutical composition comprising said at least one compound of the invention as exposed above.

The invention also relates to a method of treating a disease or disorder for which an inhibition of DHODH might be beneficial, said method comprising the administration to a subject suffering from, suspected to suffer from or at risk to suffer from said disease or disorder of at least one compound of the invention or a of composition comprising said compound. In a particular embodiment, said disease or disorder is any one of diseases or disorders as described above. In a more specific embodiment, said method comprises administering to said subject an effective amount of at least one compound of the invention.

By a "therapeutically effective amount" is meant a sufficient amount of the compound as described above to inhibit DHODH activity in the targeted cells. It will be understood, however, that the total daily usage of the compounds and compositions of the present invention will be decided by the attending physician within the scope of sound medical judgment. It is well known from the skilled in the art that specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; other drugs optionally used in combination or coincidental with the specific DHODH inhibitors of the invention which is employed; and other like factors. Standard toxicity tests and standard pharmacological assays can be used for the determination of treatment of diseases or disorders as exposed above in mammals can be used to determine the effective dosage of the compounds of the invention. For example, it is well within the skill of the art to start doses of the compound at levels lower than those required to achieve the desired therapeutic effect and to gradually increase the dosage until the desired effect is achieved. The daily dosage of the compounds of the invention may be varied over a wide range from 0.01 to 2,000 mg per adult per day. Typically, a unit dosage to contain from about 0.5 mg to about 1500 mg {e.g. about 0.5 mg to about 5 mg, about 5 mg to about 50 mg, about 50 mg to about 500 mg, about 500 mg to about 2000 mg, etc.) of said compound; for example the unit dosage contains 0.01, 0.05, 0.1, 0.5, 1.0, 2.5, 5.0, 10.0, 15.0, 25.0, 50.0, 100, 250, 500, 1000, 1,500 or 2,000 mg of said compound. Dosing schedules are also dependent upon a variety of factors as those exposed above, and will be easily determined according the afore mentioned standard pharmacological assays. It may range e.g. from one to three times a day dosing to once per week, or even every 2, 3 or 4 weeks (for example when used in conjunction with another therapy e.g. chemotherapy cycles).

The above methods, compositions or therapies may further be used in conjunction or association or combination with additional drugs or treatments. As mentioned above, in a combined therapy according to this invention, the at least two drugs and/or compounds may be administered at the same time together or separately, or sequentially. Also, the at least two drugs and/or compounds may be administered through different routes and protocols. As a result, although they may be formulated together, the drugs and/or compounds of a combination may also be formulated separately.

In a particular embodiment, additional therapies used in conjunction with compositions or compounds for use in treating cancer according to the present invention, may comprise one or more anti-cancer agent, e.g., one or more alkylating agent, purine analog, pyrimidine analog, folic acid antagonist, antitumor antibiotic, topoisomerase I or II inhibitor, histone deacetylase inhibitor, cytoskeletal disruptor (taxane), kinase inhibitor, nucleotide analogs and precursor analog, peptide antibiotic, platinum base agent, retinoid, vinca alkaloid and derivatives thereof, epitholone or derivative thereof, anti PD1, anti PDL1, anti CTLA-4, angiogenesis inhibitors or combination thereof.

Accordingly, in a more particular embodiment, compositions or compounds according to the present can be used in conjunction (i.e. either in the same formulation or separately) with at least one compound selected from cyclophosphamide, mechlorethamine, chlorambucil, melphalan, dacarbazine, nitrosoureas, temozolomide, daunorubicin, doxorubicin, epirubicin, idarubicin, mitoxantrone, valrubicin, paclitaxel, docetaxel, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, mercaptopurine, methotrexate, tioguanine, bleomycin, actinomycin, carboplatin, cisplatin, oxaliplatin, tretinoin, alitretinoin, bexarotene, vinblastine, vincristine, vindesine, vinorelbine, nivolumab, pembrolizumab, atezolizumab, avelumab, durvalumab, cemiplimab, pidilizumab, ipilimumab, AMP-224, AMP-514, PDR001, BMS-936559, CK-301, bevacizumab, thalidomide, lenalidomide, sorafenib, sunitinib, temsirolimus, axitinib, pazopanib, cabozantinib, everolimus, ramucirumab, regorafenib, vandetanib, aflibercept or ziv-aflibercept, imiquimod, dactolisib, gardiquimod, esiquimod or sumanirole or other imidazoquinoline derivative, interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a or a combination thereof. In an even more particular embodiment the invention relates to compounds of table I, or compositions comprising said compounds, used in in conjunction with at least one compound selected from cyclophosphamide, mechlorethamine, chlorambucil, melphalan, dacarbazine, nitrosoureas, temozolomide, daunorubicin, doxorubicin, epirubicin, idarubicin, mitoxantrone, valrubicin, paclitaxel, docetaxel, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, mercaptopurine, methotrexate, tioguanine, bleomycin, actinomycin, carboplatin, cisplatin, oxaliplatin, tretinoin, alitretinoin, bexarotene, vinblastine, vincristine, vindesine, vinorelbine, nivolumab, pembrolizumab, atezolizumab, avelumab, durvalumab, cemiplimab, pidilizumab, ipilimumab, AMP-224, AMP-514, PDR001, BMS-936559, CK-301, bevacizumab, thalidomide, lenalidomide, sorafenib, sunitinib, temsirolimus, axitinib, pazopanib, cabozantinib, everolimus, ramucirumab, regorafenib, vandetanib, aflibercept or ziv-aflibercept, imiquimod, dactolisib, gardiquimod, esiquimod or sumanirole or other imidazoquinoline derivative, interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a or a combination thereof. Accordingly, the invention also relates to a combination of at least one compound according to the invention with at least one compound selected from cyclophosphamide, mechlorethamine, chlorambucil, melphalan, dacarbazine, nitrosoureas, temozolomide, daunorubicin, doxorubicin, epirubicin, idarubicin, mitoxantrone, valrubicin, paclitaxel, docetaxel, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, mercaptopurine, methotrexate, tioguanine, bleomycin, actinomycin, carboplatin, cisplatin, oxaliplatin, tretinoin, alitretinoin, bexarotene, vinblastine, vincristine, vindesine, vinorelbine, nivolumab, pembrolizumab, atezolizumab, avelumab, durvalumab, cemiplimab, pidilizumab, ipilimumab, AMP-224, AMP-514, PDR001, BMS-936559, CK-301, bevacizumab, thalidomide, lenalidomide, sorafenib, sunitinib, temsirolimus, axitinib, pazopanib, cabozantinib, everolimus, ramucirumab, regorafenib, vandetanib, aflibercept or ziv-aflibercept, imiquimod, dactolisib, gardiquimod, esiquimod or sumanirole or other imidazoquinoline derivative, interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a, Interferon-γ 1b or a combination thereof. In a particular embodiment the invention relates to a combination of a DHODH inhibitor of table I with at least one compound selected from cyclophosphamide, mechlorethamine, chlorambucil, melphalan, dacarbazine, nitrosoureas, temozolomide, daunorubicin, doxorubicin, epirubicin, idarubicin, mitoxantrone, valrubicin, paclitaxel, docetaxel, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, mercaptopurine, methotrexate, tioguanine, bleomycin, actinomycin, carboplatin, cisplatin, oxaliplatin, tretinoin, alitretinoin, bexarotene, vinblastine, vincristine, vindesine, vinorelbine, nivolumab, pembrolizumab, atezolizumab, avelumab, durvalumab, cemiplimab, pidilizumab, ipilimumab, AMP-224, AMP-514, PDR001, BMS-936559, CK-301, bevacizumab, thalidomide, lenalidomide, sorafenib, sunitinib, temsirolimus, axitinib, pazopanib, cabozantinib, everolimus, ramucirumab, regorafenib, vandetanib, aflibercept or ziv-aflibercept, imiquimod, dactolisib, gardiquimod, esiquimod or sumanirole or other imidazoquinoline derivative, interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a or Interferon-γ 1b a combination thereof.

Further, the amplification of response to IFN type II is of particular advantage, when considering immune therapy to cancer. It is known in the art that immune checkpoint blockade therapy leads to upregulation of IFNγ, which in turn leads to elimination of cancer cells. Also, an increased IFNγ signature is found correlated with higher overall response rate and longer median progression free survival, in patient having received anti PDL1 therapy (Ni and Lu (2018)). As shown in the experimental section, DHODH inhibitors, and more particularly compounds of the invention amplify cellular response to IFNγ. Then, DHODH inhibitors of the invention display not only cytostatic effects but also have the capacity to boost the immune antitumoral response. Accordingly, in another particular embodiment, compositions or compounds according to the present can be used in conjunction (i.e. either in the same formulation or separately) with at least one checkpoint blockade therapy, for example at least one compound selected from nivolumab, pembrolizumab, atezolizumab, avelumab, durvalumab, cemiplimab, pidilizumab, ipilimumab or a combination thereof. In a more particular embodiment, compounds of table I are used in conjunction (i.e. either in the same formulation or separately) with at least one checkpoint blockade therapy, for example at least one compound selected from nivolumab, pembrolizumab, atezolizumab, avelumab, durvalumab, cemiplimab, pidilizumab, ipilimumab or a combination thereof. In an even more particular embodiment such combinatorial treatment can further be associated with other anticancer agent.

Combination therapies are particularly advantageous as they can lead to expect an increased efficacy in treating the disease, and also in some instance, a lowering of doses of at least one of the components of said combinatorial treatment while obtaining the same or even a better therapeutic effect when compared to each of the component when used alone (through an additive or a synergistic effect). Therefore, combinatory therapies of the invention allow to consider a possible lowering of dosages, thereby diminishing or even avoiding adverse effects, which is of particular interest in the case of anti-cancer drugs such as chemotherapeutic agents. Determining an actual synergistic effect from combinations of the invention is well known by the skilled in the art and further described in Foucquier and Guedj (2015).

As shown in the experimental section, compounds of the invention are found to allow the amplification of cellular response to type I and II IFNs. This leads to expect an increase in efficacy of treatments based upon the use interferons or agonists thereof, for example, treatment of some cancer (e.g. malignant melanoma, sarcoma, leukaemia and lymphomas), viral infections (e.g. hepatitis B, C or E), autoimmune disorders (e.g. multiple sclerosis). Furthermore, this leads also to consider a possible lowering of doses of interferon-based treatment, thereby diminishing or even avoiding adverse effects associated with their use.

Accordingly, in a particular embodiment, compositions or compounds according to the present invention can be used in conjunction (i.e. either in the same formulation or separately) with at least one interferon type I or II agonist, for example a compound selected from imiquimod, dactolisib, gardiquimod, esiquimod or sumanirole or other imidazoquinoline derivative, interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a, Interferon-γ 1b or a combination thereof.

In another embodiment, the invention relates to a method of treating cancer comprising the step of:
i) Determining the sensibility of the tumor of a subject to a DHODH inhibitor according to the invention,
ii) implementing the treatment of the subject with said DHODH inhibitor, if the tumor is found to be sensitive to this compound.

In a particular embodiment step i) of determining the sensibility of the tumor of a subject to a DHODH inhibitor according to the invention comprises incubating cells from said tumor with a DHODH inhibitor of the invention and measuring proliferation of these cells. Methods for measuring cell proliferation are known from the skilled in the art.

In another particular embodiment step i) of determining the sensibility of the tumor of a subject to a DHODH inhibitor according to the invention comprises identifying marker(s) in these cells of said tumor, said marker(s) known to be associated with such a sensibility. Said marker can be either genetic, metabolic or proteomic markers. In a particular embodiment said marker is related to DHODH gene or protein.

In a yet particular embodiment, step i) is performed *in vitro*, and cells from the tumor are obtained from a tumor biopsy, or from a sample of a total or partial resection of said tumor.

In an even more particular embodiment, compounds of the invention provide valuable tools for *in vitro* assays necessitating the inhibition of dihydroorotate dehydrogenase (DHODH) inhibitor. An object of the invention thus relates to the use of a compound according to the invention as dihydroorotate dehydrogenase (DHODH) inhibitor in an *in vitro* assay.

### EXAMPLES

The following abbreviations have been used:
- ESI: : Electrospray ionisation
- Et: : Ethyl (CH₂CH₃)
- IFN: : Interferon
- HRMS: : High Resolution Mass Spectrometry
- MS: : Mass Spectroscopy
- mp: : melting point
- NMR: : Nuclear Magnetic Resonance
- TMS: : Tetramethylsilane

The examples that follow illustrate the invention without limiting its scope in any way.

### 1. Example 1, Generic synthetic route of 5-cyclopropyl- [3,4-c]pyridine-3,4(1H,5H)-dione 1,1' substituted derivatives of the invention

Compounds of the invention can be prepared by the following reaction

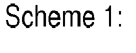

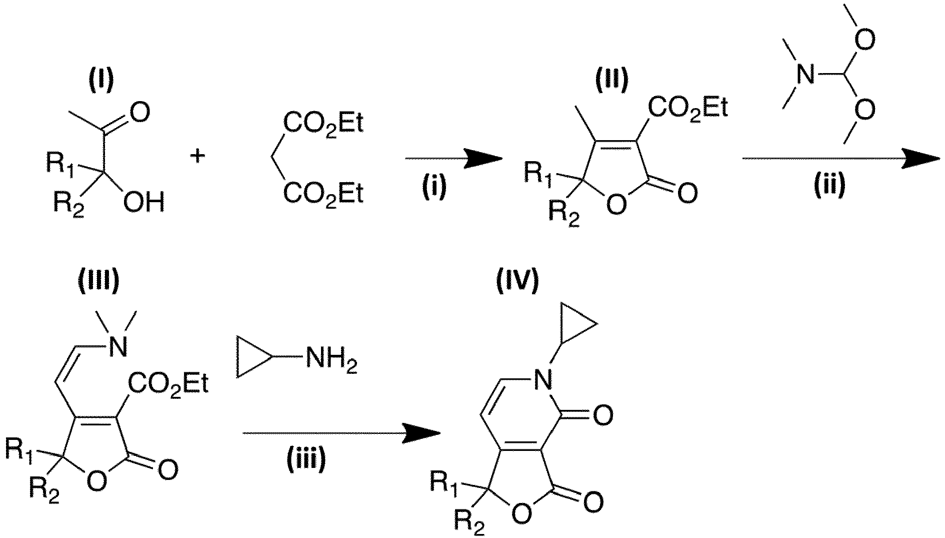

Step i) To obtain compound (II), a solution of 0.1 mol α-ketoalcohol (compound I) and 0.11 mol diethylmalonate in 50 mL of anhydrous ethanol with the presence of catalytic amount of sodium ethylate is refluxed for 20 hours and solvent is removed under the reduced pressure. Cooled reaction mixture is neutralized with diluted hydrochloric acid (6N) and extracted with diethyl ether (3x30 mL). Combined extracts are dried with magnesium sulfate and the solvent is removed under reduced pressure. The residue is dried under vacuum and then used without purification for the second step.

Step ii) A mixture of compound II (10 mmol) and DMF/DMA (12 mmol) in an anhydrous xylene (12 mL) was refluxed for 3 h, then cooled and 5 mL of diethyl ether was added. The precipitated crystals are filtered, washed with diethyl ether and dried under vacuum to obtain compound III

Step iii) Compound III (6mmol) is put to react directly with cyclopropylamine (12 mmol) at 40° for 15 h. Although Compound IV is obtained as soon as 10 hours of reaction, inventors have noticed that the maximum yield is reached around 15 hours of reaction. The mixture is cooled to room temperature then 10 mL of diethyl ether is added to obtain after filtration and washing with diethyl ether, precipitated crystals of 5-cyclopropyl- [3,4-c]pyridine-3,4(1H,5H)-dione 1,1' substituted derivatives of the invention.

The skilled in the art will know without any burden or difficulty how to adapt this procedure to obtain any of the 5-cyclopropyl- [3,4-c]pyridine-3,4(1H,5H)-dione 1,1' substituted derivatives of the invention.

### 2. Example 2, synthesis of compounds 1-4

### Precursor compound (I)

Intermediate compounds I are commercially available (providers are, e.g. Alfa Aesar or Sigma Aldrich) or even can be easily homemade without any burden; for example, intermediate compounds (I) for the synthesis of compounds 1 to 7 according to the invention are indicated in table 2; those corresponding to other compounds of the invention can readily be deduced.

**Table 2**

| *Compound of the invention* | *Intermediate compound (I)* | *Compound of the invention* | *Intermediate compound (I)* |
|---|---|---|---|
| *Compound 1* | 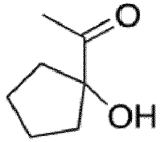 | *Compound 5* | 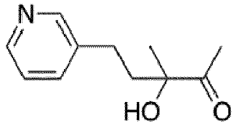 |
| *Compound 2* | 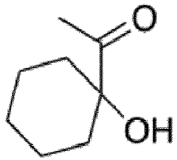 | *Compound 6* | 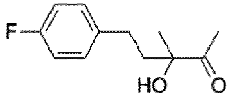 |
| *Compound 3* | 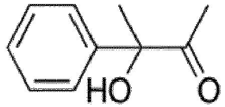 | *Compound 7* | 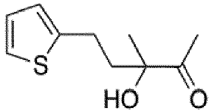 |
| *Compound 4* | 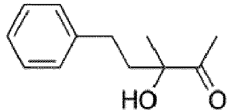 | | |

### Compounds characterization

¹H NMR and ¹³C NMR spectra were recorded in deuterated DMSO-d6 at ambient temperature using a 500 MHz NMR spectrometer equipped with a 5 mm cryogenically cooled probe ¹H/¹³C/²D optimized for carbon-13 detection with z-gradients. The chemical shifts were reported in ppm downfield from TMS. ESI and HRMS analyses were conducted using a Thermo Scientific High Resolution Exactive Orbitrap Mass spectrometer.

Results for compound 1-4 are given below :

### Compound 1 (5'-cyclopropyl-3'H-spiro[cyclopentane-1,1'-furo[3,4-c]pyridine]-3',4'(5'H)-dione).

Yield= 72 %, mp 190-192 °C.
¹H NMR, δ(ppm): 0.89 (m, 2H), 1.02 (m, 2H), 1.89 (m, 6H), 2.09 (m, 2H), 3.33 (m, 1H), 6.55 (d, J = 6.9 Hz, 1H), 8.03 (d, J = 6.9 Hz, 1H).
¹³C NMR, δ(ppm): 6.5 (2CH₂), 24.5 (2CH₂), 32.1, 37.9 (2CH₂), 91.8, 98.2, 110.9, 146.1, 157.5, 166.2, 169.5.
MS [ESI+, MeOH]: [M+H]⁺ 246.11 (48%), [M+Na]⁺ 268.09 (52%), [2M+Na]⁺ 513.20 (100%). HRMS [ESI+, MeOH]: calcd for C₁₄H₁₆O₃N [M+H]⁺ 246.1125 found 246.1128.

### Compound 2 (5'-cyclopropyl-3'H-spiro[cyclohexane-1,1'furo[3,4-c]pyridine]-3',4'(5'H)-dione)

Yield 76 %, mp 235-236 °C.
¹H NMR, δ(ppm): 0.88 (m, 2H), 1.02 (m, 2H), 1.36 (m, 1H), 1.57 (m, 4H), 1.72 (m, 3H), 1.89 (dt, J = 4.0, 13.1 Hz, 2H), 3.3 (m, 1H), 6.56 (d, J = 6.8 Hz, 1H), 8.01 (d, J = 6.8 Hz, 1H).
¹³C NMR, δ(ppm): 6.4 (2CH2), 21.6 (2CH2), 23.8, 32.1, 33.7 (2CH2), 83.4, 98.4, 110.1, 146, 157.9, 166.3, 171.3.
MS [ESI+, MeOH]: [M+H]+ 260.1 (70%), [2M+Na]+ 541.2(100%). El. anal. calcd. for C15H17NO3 : C 69.48; H 6.61; N 5.40; Found: C 69.66; H 6.63; N 5.40.

### Compound 3 (5-cyclopropyl-1-methyl-1-phenylfuro[3,4-c]pyridine-3,4(1H,5H)-dione)

Yield 73 %, mp 186 °C.
¹H NMR, δ(ppm) 7,88 (d,J=6,9Hz, 1H), 7,50-7,24(m,5H),6,42(d,J =6,48Hz,1H),2,55-2,44(m,1H), 1,96 (s,3H), 1,15-0,81 (m,4H).
¹³C NMR, δ(ppm): (75MHz DMSO),170.03, 165.52, 159.42, 157.07, 145.39, 139.00, 128.23, 127.81,124.61, 95.48, 40.33, 40.05, 39.77, 39.49,39.22, 38.94, 38.67, 31.71,24.92, 6.38, 6.31
MS [ESI+, MeOH]: [M+H]⁺ 282.11 (32%), [M+Na]⁺ 304.09 (76%), [2M+Na]⁺ 585.20 (100%).
HRMS [ESI+, MeOH]: calcd for C₁₇H₁₆O₃N [M+H]⁺ 282.1125 found 282.1125.

### Compound 4 (5-cyclopropyl-1-methyl-1-phenethylfuro[3,4-c]pyridine-3,4(1H,5H)-dione)

Yield 61 %, mp 116 °C.
¹H NMR, δ(ppm)(300MHz,DMSO) 7,86(d,J=6.8Hz, 1H), 7.22-7.14 (m,2H),7.12-7.04(m, 3H),6,41 (d,J=6.8 Hz,1H),3.40-3.30 (m,1H),2.66-2.52(m,1H),2.40-2.07 (m,3H),1.59(s,3H),,1.16-1.06(m,2H),0.98-o.83(m,2H).
¹³C NMR, δ(pp m)(75 MHz,DMSO) 169.64,165.37,156.99,144.92, 140.16, 127.75, 127.64,,125.30,111.25,95.45,40.05,39.77, 39.49,39.24,38.94, 31.63, 29.00,24.36, 6.34, 6.31
MS [ESI+, MeOH]: [M+H]⁺ 310.14 (44%), [M+Na]⁺ 332.13 (92%), [2M+Na]+ 641.26 (100%)111.
HRMS [ESI+, MeOH]: calcd for C₁₉H₂₀O₃N [M+H]⁺ 310.1438 found 310.1437.

### 3. Example 3, Pyrimidine synthesis inhibition by compounds of the invention

### Methods

### HEK-293 cell treatment.

Cells were cultured at 37°C and 5% CO2 in Dulbecco's modified Eagle's Medium (DMEM; Sigma-Aldrich) containing 10% fetal calf serum (FCS), penicillin and streptomycin. Nucleoside/nucleotide quantification in HEK-293 cells was performed as previously described in by Lucas-Hourani *et al.* (2017). Briefly, cells were plated in 6-well plates at 1x10⁶ cells per well and after 24 h, several concentrations of compounds of the invention (diluted in DMSO) or DMSO alone was added.

### Purine (G/A) and pyrimidine (C/U) quantification by HPLC-coupled spectrophotometry.

One day later, cells were washed with PBS and cellular pellets were deproteinized by adding an equal volume of 6% perchloric acid. After 10 min of incubation on ice and clearing of the extracts by centrifugation (13,000 rpm for 10 min at 4°C), supernatants were supplemented with bidistilled water (V/V) and neutralized by the addition of 2 M Na₂CO₃. Extracts were analyzed by HPLC onto a C18 Supelco 5-µm (250 by 4.6 mm) column (Sigma) at 45°C using a mobile phase delivered at a flow rate of 1 mL/min. Buffer A contained 10 mM tetrabutylammonium hydroxide, 10 mM KH₂PO₄, and 0.25% MeOH and was adjusted to pH 6.9 with 1 M HCl. Buffer B contained 5.6 mM tetrabutylammonium hydroxide, 50 mM KH₂PO₄, and 30% MeOH and was neutralized to pH 7.0 with 1 M NaOH. The following stepwise gradient elution program was used: A to B at 60:40 at 0 min, 40:60 at 30 min, and 40:60 at 60 min. Products were monitored spectrophotometrically at 254 nm with a diode array detector (PDA) and quantified by integration of the peak absorbance area. Calibration curves were generated with reference nucleosides. Finally, raw data were normalized to the total number of viable cells present in the well for taking into account minor differences between culture conditions.

Three independent experiments were performed for each point.

### Cell viability assays

Cells were treated with DMSO or compounds of the invention at different concentrations, and the number of viable cells was determined using the CellTiter-Glo reagent after 24, 48 and 72 h of culture. Cellular viability was determined by quantification of ATP in culture wells using the CellTiter-Glo assay (Promega).

Two independent experiments were performed. Results were analyzed using one-way analysis of variance (ANOVA) with Bonferroni's post hoc test.

### Results

Nucleoside levels measured in HEK cells treated or not treated with compounds of the invention are shown in Figure 1 A.

Pyrimidine (U and C) levels collapsed in cells treated with compounds of the invention, whereas purine levels were unaffected (G and A), thus establishing that compounds of the invention interfere with pyrimidine homeostasis. Similar results were previously observed with DD778, a known DHODH inhibitor (not shown; Lucas-Hourani *et al.* (2017)).

Furthermore, compounds of the invention are found able to block cellular proliferation, a phenotype that is also associated to DHODH inhibition. Indeed, they efficiently inhibited the proliferation of HEK-293 cells when monitored over three days (Figure 1B), this inhibition is probably linked to the drop in pyrimidine levels, and thus make the compounds of the invention useful for treating diseases related to uncontrolled proliferation of cells which particularly need *de novo* bases synthesis, e.g. cancer or immune diseases. In line with this cytostatic effect, inventors have found that treatment with compounds of the invention is associated the induction of a DNA damage response associated to the inhibition of pyrimidine biosynthesis and the subsequent halt of replication forks (not shown).

### 4. Example 4, Amplification of cellular response to IFN-α

It is known from the literature that DHODH inhibitors amplify cellular response to IFN-I. It was made use from the reporter system as described in Lucas-Hourani et al. (2013) or Yeo *et al.* (2015) to assess the amplification of IFN-I response through incubation with compounds of the invention, and to define common structural features of DHODH inhibitors of the invention.

### Methods

### Reporter system and cell culture

Cells were cultured at 37°C and 5% CO₂ in Dulbecco's modified Eagle's Medium (DMEM; Sigma-Aldrich) containing 10% fetal calf serum (FCS), penicillin and streptomycin. Experiments were performed on HEK-293 cells stably transfected with the ISRE-Luciferase reporter gene (ISRE-Luc), with or without NanoLuc as an additional reporter gene (Lucas-Hourani *et al*., 2013; Hayek *et al*., 2019).

Reporter cells were incubated with increasing concentrations of candidates DHODH inhibitors diluted in DMSO in the presence or not of recombinant IFN-α (150 IU/mL). Recombinant IFN-α was from Sigma-Aldrich or PBL Assay Science. Luciferase activity was quantified 24 h later.

To confirm that modulation of IFN-α is related to DHDOH inhibition by compounds of the invention, the same experiments were performed by adding either uridine (pyrimidine derivated nucleoside), dihydroorotate (DHO, the substrate od DHODH in pyrimidine *de novo* biosynthesis pathway) or orotate (the product of ubiquinone-mediated oxidation of DHO catalysed by DHODH) together with compound of the invention. uridine, orotate, and DHO are all from Sigma Aldrich.

### Luciferase expression assay

Firefly luciferase expression in culture wells was measured using the Bright-Glo (Promega) or Britelite plus reagents (PerkinElmer), according to the manufacturer's recommendations. For each concentration, three independent experiments were performed.

Compounds were tested for their ability to boost cellular response to IFN-α using ISRE-Luc reporter cells. A dose-response ranging from 0.8 to 150 µM was established for each compound, and corresponding pEC50s (i.e. -log10 of the half maximal effective molar concentrations) where determined. Compounds with a pEC50<3.8 are inactive.

### Results

Among the several compounds tested, only compounds of the invention are found efficient in amplifying cellular response to IFN-α response as shown in Figure 2 and table 3, whereas no luciferase activity was detected in absence of IFN-α.

Notably, comparison of the pEC50s of the different tested compounds clearly shows the importance of furopyridine scaffold (compare compound 37 with compounds 1-4), of the 5-cyclopropyl group on the azote of furopyridine (compare compound 2 with compounds 38-42), as well as of the two keto groups (compare compound 2 with compound 36). Study of structure/activity relationship also shows that a hydrophobic ring at position 1 of the furopyridine scaffold, such as in compounds 1-3 provide efficient DHODH inhibitors, as well as when this hydrophobic ring is linked to the furopyridine scaffold through an alkyl chain (compound 4). Other compounds of the invention such as compounds 5-33 are also found effective DHODH inhibitor regarding their effect in amplifying response to IFN-α

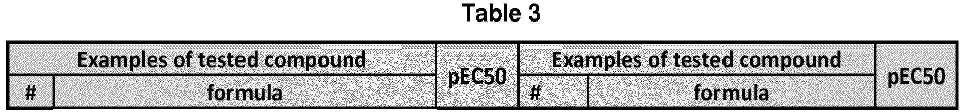

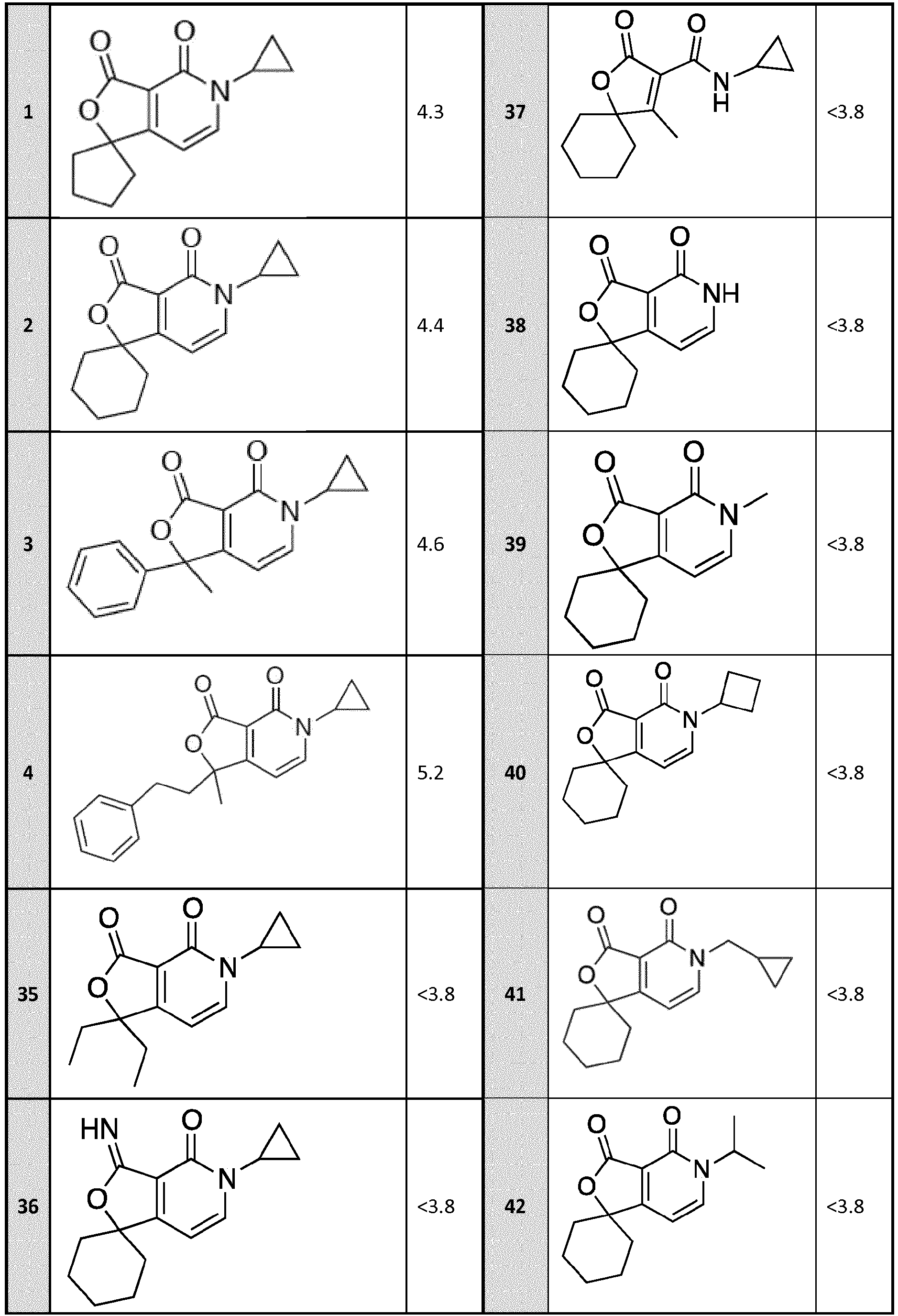

Compounds of the invention thus share the ability to amplify response to IFN-α. Furthermore, uridine (Figure 3A) and orotate (Figure 3B), downstream compounds in the *de novo* pyrimidine biosynthesis pathway, are shown to revert amplification effect whereas DHO (substrate of DHODH) does not, thereby confirming that compound of the invention are inhibitors of DHODH within the pyrimidine biosynthesis pathway. These results are in line with the drop in pyrimidine content of the cells as described in example 3 and Lucas-Hourani *et al.* (2013).

### 5. Example 5, Amplification of cellular response to type II IFN

### Methods

### Reporter system and cell culture

Reporter system, cell culture and luciferase assay are performed as exposed above. HEK-293 cells with the ISRE-Luc reporter gene were treated with DMSO or compounds of the invention in the absence or presence of recombinant IFN-γ (Figure 4A ; Roussel Uclaf or Milteny Biotech, 150 IU/mL).

The same experiment was performed but using different dilutions ranging from 1/8 to 1/64 of conditioned supernatants from activated PBMCs, instead of recombinant IFN-γ. Human peripheral blood mononuclear cells (PBMC) from one healthy donor were isolated by density centrifugation with Lymphoprep medium (StemCell Technologies) from leucocytes concentrates obtained when plateletpheresis was performed (Etablissement Français du Sang; Paris; France). To activate PBMCs, cells were treated for 24 h with the TLR7/8 ligand R848 at 5 µg/mL (Sigma Aldrich) and supernatants were harvested. R848 is known to induce expression of several cytokines, within which IFN-γ which is found at high levels in PBMC supernatants in comparison with type I IFNs (Table 4).

**Table 4. Cytokine expression levels in culture supernatants of human PBMC activated with R848.**

| **Cytokine** | **PBMC alone** | **PBMC + R848** |
|---|---|---|
| IL-1β | ND | 12 ng/mL |
| IL-6 | ND | 65 ng/mL |
| TNF-α | ND | 11 ng/mL |
| IP10 | 0.002 ng/mL | 0.028 ng/mL |
| IFN-λ1 | 0.002 ng/mL | 0.016 ng/mL |
| IL-8 | 0.149 ng/mL | 214 ng/mL |
| IL-12p70 | ND | 0.009 ng/mL |
| IFN-α2 | ND | 0.097 ng/mL |
| IFN-λ2/3 | ND | ND |
| GM-CSF | ND | 0.043 ng/mL |
| IFN-β | ND | 0.015 ng/mL |
| IL-10 | ND | 0.283 ng/mL |
| IFN-**γ** | **ND** | **4.2 ng/mL** |

| | | |
|---|---|---|
| *ND :"Not Detected".* | | |

### Results

Compounds of the invention are also found efficient in amplifying cellular response to IFN-γ response (an increase of more than 4-fold in Luciferase expression Is noticed) as shown in Figure 4A, whereas no luciferase activity was detected in absence of IFN-γ. Such an effect is also observed when reporter cells are incubated with conditioned supernatants of activated PBMC Figure 4B.

These results highlight for the first time that the benefit of DHODH inhibitors, and more particularly DHODH inhibitors of the invention, relies not only on their cytostatic effects but also on their capacity to boost the antitumoral response. Indeed, IFN-γ is massively produced by activated NK cells and CD8+ T lymphocytes and is known for playing a key role in the antitumoral response. Indeed, it is known in the art that immune checkpoint blockade therapy led to upregulation of IFNγ and in turn clearance of tumor cells (Ni and Lu, 2018).

### 6. Example 6, measurement of cell proliferation

### In vitro assays

Numerous assays are available to test the inhibition of cancer cell line or primary tumor cell, or even immune cell proliferation by DHODH inhibitor of the invention for example, in a non limitative way, CellTiter-Glo assay or the MTT assay.

### CellTiter-Glo assay

Cells are treated with DMSO alone or compounds of the invention at different concentrations (diluted in DMSO), and the number of viable cells is determined using the CellTiter-Glo reagent after 24, 48 and 72 h of culture. The number of viable cells is determined by quantification of ATP in culture wells using the CellTiter-Glo assay (Promega).

### MTT assay

In this assay, cellular viability and/or proliferation is measured by assessing the reduction of tetrazolium dye MTT 3-(4,5-dimethylthiazol-2-yl)-2,5-diphenyltetrazolium by NAD(P)H-dependent cellular oxidoreductase enzyme, which results in the production and accumulation of an insoluble purple product, formation of which is easily measurable by measuring absorbance at 570 nm. Briefly, cell line or tumor cells are treated with various concentration of compound of the invention. Non treated cells are included as negative control. Proliferation and number of viable cells is determined each day during 3 days, by measuring the absorbance at 570nm (i.e. the accumulation of reduction product of MTT).

### In vivo assays

AML cancer is known to be sensitive to DHODH inhibitors. THP1, HL60, and MOLM13 are well known AML cancer cell lines available at ATCC. Xenograft in experiments are performed in female NOD/SCID (NOD.CB17-Prkdcscid) recipient mice 6-8 weeks of age to measure the inhibition of tumor growth by the compound of the invention. THP1, HL60, or MOLM13 Cells (5. 10⁶) were implanted subcutaneously in a Matrigel matrix under the right flank of each mouse and are allowed to grow to the pre-specified size of 40 mm². Subcutaneous tumor volumes were determined by caliper measurement. Mice are then treated with vehicle or candidate compound via i.p. injection in a final volume of 250 µl (10 ml/kg). Candidate compound are given at different doses ranging from 1 mg/kg daily or to 20 mg/kg every day. The experiments are stopped when tumors in the control group reach the pre-specified size of 150 mm2. Tumor volume in treated or not treated animal and also function of dose is then compared.

### 7. Conclusion

Experimental data thus show that compounds of the invention are effective DHODH inhibitors which are able to amplify cellular response to class I and II interferons, and also to inhibit *de novo* pyrimidine synthesis. These compounds are thus valuable candidates for treating any diseases for which inhibition of cell proliferation and / or amplification of response to type I and II IFNs is of some interest, namely proliferative disorders like cancers and more specifically acute myeloid leukemia, immune or autoimmune diseases, viral infections and the like. Compounds of the invention thus provide valuable therapeutics tools to be used either alone or in a combination with other therapies like recombinants interferons, chemotherapeutic agents, check point inhibitors or other cancer immunotherapies.

### REFERENCES

Foucquier, J,and Guedj, M (2015). Analysis of drug combinations: current methodological landscape. Pharmacol Res Perspect. 3(3): e00149
Hayek, S, Bekaddour, N, Besson, L, Alves de Sousa, R, Pietrancosta, N, Viel, S,Smith, N, Jacob, Y, Nisole, 5, Mandal, R, Wishart, DS, Walzer, T, Herbeuval, JP, Vidalain PO (2019). Identification of Primary Natural Killer Cell Modulators by Chemical Library Screening with a Luciferase-Based Functional Assay. SLAS Discov. 24(1):25-37.
Lucas-Hourani, M., Dauzonne, D., Jorda, P., Cousin, G., Lupan, A., Helynck, O., Caignard, G., Janvier, G., Andre-Leroux, G., Khiar, S., et al. (2013). Inhibition of pyrimidine biosynthesis pathway suppresses viral growth through innate immunity. PLoS Pathog. 9.
Lucas-Hourani, M., Dauzonne, D., Munier-Lehmann, H., Khiar, S., Nisole, S., Dairou, J., Helynck, O., Afonso, P.V., Tangy, F., and Vidalain, P.-O. (2017). Original Chemical Series of Pyrimidine Biosynthesis Inhibitors That Boost the Antiviral Interferon Response. Antimicrob. Agents Chemother. 61.
Madak, JT, Bankhead, A, Cuthbertson, CR, Showalter, HD, Neamati, N, (2019). Revisiting the role of dihydroorotate dehydrogenase as a therapeutic target for cancer. Pharmacol Ther. 195:111-131.
Ni L. and Lu J. (2018) Interferon gamma in cancer immunotherapy. Cancer Medicine. ;7:4509-4516.
Sykes, DB, (2018). The emergence of dihydroorotate dehydrogenase (DHODH) as a therapeutic target in acute myeloid leukemia Expert Opin Ther Targets. 22(11):893-898.
Yeo, K.L., Chen, Y.-L., Xu, H.Y., Dong, H., Wang, Q.-Y., Yokokawa, F., and Shi, P.-Y. (2015). Synergistic suppression of dengue virus replication using a combination of nucleoside analogs and nucleoside synthesis inhibitors. Antimicrob. Agents Chemother. 59, 2086-2093..

## Claims

1. A compound of general formula I, its salts, solvates and / or stereoisomers, 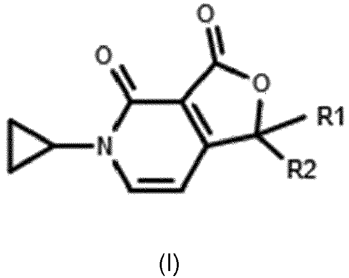
- in which R1 and R2 each represent, independently from each other a group selected from
∘ a (C1-C8)alkyl or heteroalkyl,
∘ a (C3-C6) cycloalkyl or a 3- to 6- membered heterocycloalkyl,
∘ a (C3-C6) cycloalkenyl or 3- to 6- membered heterocycloalkenyl,
∘ a phenyl, a tolyl, or a phenyl-,cyclohexyl-or heterocycle-substituted phenyl,
∘ a cycloalkyl-(C1-C8)alkyl or a heterocycloalkyl-(C1-C8)alkyl,
∘ a cycloalkenyl-(C1-C8)alkyl or a heterocycloalkenyl-(C1-C8)alkyl, or
∘ an aryl (C1-C8)alkyl, or a heteroaryl (C1-C8)alkyl,
With the proviso that at least one of R1 or R2 is a group selected from :
∘ a (C3-C6) cycloalkyl or a 3- to 6- membered heterocycloalkyl,
∘ a (C3-C6) cycloalkenyl or 3- to 6- membered heterocycloalkenyl,
∘ a phenyl, a tolyl, or a phenyl-,cyclohexyl-or heterocycle-substituted phenyl,
∘ a cycloalkyl-(C1-C8)alkyl or a heterocycloalkyl-(C1-C8)alkyl,
∘ a cycloalkenyl-(C1-C8)alkyl or a heterocycloalkenyl-(C1-C8)alkyl, or
∘ an aryl (C1-C8)alkyl, or a heteroaryl (C1-C8)alkyl,
or
- R1 and R2 form together a bond or a (C3-C8) cycloalkyl, a 3- to 8- membered heterocycloalkyl, a (C3-C8) cycloalkenyl or a 3- to 8- membered heterocycloalkenyl.

2. The compound according to claim 1, its salts, solvates and / or stereoisomers, in which:
- R1 is a (C1-C8) alkyl, and
- R2 is a group selected from:
∘ a (C3-C6) cycloalkyl or a 3- to 6- membered heterocycloalkyl,
∘ a (C3-C6) cycloalkenyl or 3- to 6- membered heterocycloalkenyl,
∘ a cycloalkyl-(C1-C8)alkyl or a heterocycloalkyl-(C1-C8)alkyl,
∘ a cycloalkenyl-(C1-C8)alkyl or a heterocycloalkenyl-(C1-C8)alkyl, or
∘ an aryl (C1-C8)alkyl, or a heteroaryl (C1-C8)alkyl.

3. The compound according to claim 1, wherein:
- R1 is a (C1-C8) alkyl, and
- R2 is a group selected from a phenyl, napthyl-(C1-C8) alkyl, indolyl-(C1-C8) alkyl, pyrrolyl-(C1-C8) alkyl, furanyl-(C1-C8) alkyl, aryl-(C1-C8) alkyl, heteroaryl-(C1-C8) alkyl, cyclopentadienyl-(C1-C8) alkyl, heterocyclopentandienyl-(C1-C8) alkyl, said group being optionally substituted by one or more group selected independently from (C1-C6) alkyl, halogen atom or N, O, S, methoxy or nitro groups.
its salts, solvates and / or stereoisomers.

4. The compound according to claim 1, wherein R1 and R2 form together a (C3-C8) cycloalkyl, optionally substituted by one or more group selected independently from (C1-C6) alkyl, halogen atom, amine, hydroxy, thiol, methoxy or nitro group, its salts, solvates and / or stereoisomers.

5. The compound according to claim 1, selected from:
5'-cyclopropyl-3'H-spiro[cyclopentane-1,1'-furo[3,4-c]pyridine]-3',4'(5'H)-dione,
5'-cyclopropyl-3'H-spiro[cyclohexane-1,1'-furo[3,4-c]pyridine]-3',4'(5'H)-dione,
5-cyclopropyl-1-methyl-1-phenylfuro[3,4-c]pyridine-3,4(1H,5H)-dione,
5-cyclopropyl-1-methyl-1-phenethylfuro[3,4-c]pyridine-3,4(1 H,5H)-dione,
5-cyclopropyl-1-methyl-1-(2-(pyridin-3-yl)ethyl)furo[3,4-c]pyridine-3,4(1H,5H)-dione,
5-cyclopropyl-1-(4-fluorophenethyl)-1-methylfuro[3,4-c]pyridine-3,4(1H,5H)-dione,
5-cyclopropyl-1-methyl-1-(3-(thiophen-2-yl)propyl)furo[3,4-c]pyridine-3,4(1H,5H)-dione,
5-cyclopropyl-1-methyl-1-(3-phenylpropyl)furo[3,4-c]pyridine-3,4(1H,5H)-dione,
5-cyclopropyl-1-(3-(4-fluorophenyl)propyl)-1-methylfuro[3,4-c]pyridine-3,4(1H,5H)-dione,
5-cyclopropyl-1-methyl-1-(3-(pyridin-3-yl)propyl)furo[3,4-c]pyridine-3,4(1H,5H)-dione,
5-cyclopropyl-1-methyl-1-(3-(pyridin-4-yl)propyl)furo[3,4-c]pyridine-3,4(1H,5H)-dione,
5-cyclopropyl-1-methyl-1-(3-(pyridin-2-yl)propyl)furo[3,4-c]pyridine-3,4(1H,5H)-dione,
5-cyclopropyl-1-methyl-1-(3-(naphthalen-2-yl)propyl)furo[3,4-c]pyridine-3,4(1H,5H)-dione,
5-cyclopropyl-1-methyl-1-(3-(naphthalen-1-yl)propyl)furo[3,4-c]pyridine-3,4(1H,5H)-dione,
5-cyclopropyl-1-methyl-1-(3-(1-methyl-3a,7a-dihydro-1H-indol-3-yl)propyl)furo[3,4-c]pyridine-3,4(1H,5H)-dione,
5-cyclopropyl-1-methyl-1-(3-(1-methyl-1H-pyrrol-3-yl)propyl)furo[3,4-c]pyridine-3,4(1H,5H)-dione,
5-cyclopropyl-1-(3-(furan-3-yl)propyl)-1-methylfuro[3,4-c]pyridine-3,4(1H,5H)-dione,
5-cyclopropyl-1-methyl-1-(3-(thiophen-3-yl)propyl)furo[3,4-c]pyridine-3,4(1H,5H)-dione,
1-benzyl-5-cyclopropyl-1-methylfuro[3,4-c]pyridine-3,4(1H,5H)-dione,
5-cyclopropyl-1-methyl-1-(4-phenylbutyl)furo[3,4-c]pyridine-3,4(1H,5H)-dione,
5-cyclopropyl-1-methyl-1-(3-phenylbutyl)furo[3,4-c]pyridine-3,4(1H,5H)-dione,
5-cyclopropyl-1-methyl-1-(2-(pyridin-4-yl)ethyl)furo[3,4-c]pyridine-3,4(1H,5H)-dione,
5-cyclopropyl-1-methyl-1-(2-(pyridin-3-yl)ethyl)furo[3,4-c]pyridine-3,4(1H,5H)-dione,
5-cyclopropyl-1-methyl-1-(2-(pyridin-2-yl)ethyl)furo[3,4-c]pyridine-3,4(1H,5H)-dione,
5-cyclopropyl-1-methyl-1-(2-(naphthalen-2-yl)ethyl)furo[3,4-c]pyridine-3,4(1H,5H)-dione,
5-cyclopropyl-1-methyl-1-(2-(naphthalen-1-yl)ethyl)furo[3,4-c]pyridine-3,4(1H,5H)-dione,
5-cyclopropyl-1-methyl-1-(2-(1-methyl-3a,7a-dihydro-1H-indol-3-yl)ethyl)furo[3,4-c]pyridine-3,4(1H,5H)-dione,
5-cyclopropyl-1-methyl-1-(2-(thiophen-2-yl)ethyl)furo[3,4-c]pyridine-3,4(1H,5H)-dione,
5-cyclopropyl-1-(2-(furan-2-yl)ethyl)-1-methylfuro[3,4-c]pyridine-3,4(1H,5H)-dione,
5-cyclopropyl-1-methyl-1-(2-(1-methyl-1H-pyrrol-2-yl)ethyl)furo[3,4-c]pyridine-3,4(1H,5H)-dione,
5-cyclopropyl-1-(4-fluorophenethyl)-1-methylfuro[3,4-c]pyridine-3,4(1H,5H)-dione,
5-cyclopropyl-1-(4-methoxyphenethyl)-1-methylfuro[3,4-c]pyridine-3,4(1H,5H)-dione, and
5-cyclopropyl-1-methyl-1-(4-nitrophenethyl)furo[3,4-c]pyridine-3,4(1H,5H)-dione,
or its salts, solvates and / or stereoisomers.

6. A pharmaceutical composition comprising at least one compound according to anyone of claims 1 to 5.

7. The compound according to anyone of claims 1 to 5 for its use as a medicament.

8. The compound according to anyone of preceding claims 1 to 5 for its use in treating or preventing a disease responsive to pyrimidine *de novo* biosynthesis inhibitors.

9. The compound according to anyone of preceding claims 1 to 5 for its use in treating or preventing a proliferative disorder.

10. The compound according to anyone of claims 1 to 5 for its use in treating a disease selected from:
- cancer, preferably selected from small and large intestine cancer, lung cancer, e.g. *RAS*/*LKB1* double-mutant lung cancer, hepatocellular carcinoma (HCC), haematopoietic or lymphoid cancer, Acute Myeloid Leukemia (AML), ovary cancer, PTEN deficient or triple negative breast cancer, KRAS mutant pancreatic cancer, malignant melanoma, e.g. BRAF^{V600E}-mutant melanoma,
- autoimmune diseases or immune and inflammatory diseases, e.g. psoriasis, multiple sclerosis, relapsing remitting multiple sclerosis, rheumatoid arthritis, ulcerative colitis or Crohn's disease, lupus erythematosus, graft versus host disease, graft/transplant rejection. or
- viral infections, e.g. infection to influenza virus, dengue virus, chikungunya virus, human cytomegalovirus, human respiratory syncytial virus, measles virus, coxsackieviruses, rotavirus, Ebola virus, hepatitis B, C or E viruses.

11. The compound for the use according to claim 10, wherein said disease is cancer and wherein said use further comprises the combined or separated, simultaneous or sequential, use of at least one chemotherapeutic agent and/or immune checkpoint inhibitor and/or Interferon type I or II agonist.

12. The compound for the use according to anyone of claims 10 to 11 wherein said use further comprises the combined or separated, simultaneous or sequential use of at least one compound selected from cyclophosphamide, mechlorethamine, chlorambucil, melphalan, dacarbazine, nitrosoureas, temozolomide, daunorubicin, doxorubicin, epirubicin, idarubicin, mitoxantrone, valrubicin, paclitaxel, docetaxel, vorinostat, romidepsin, irinotecan, topotecan, etoposide, teniposide, tafluposide, bortezomib, erlotinib, gefitinib, imatinib, vemurafenib, vismodegib, azacitidine, azathioprine, capecitabine, cytarabine, doxifluridine, fluorouracil, gemcitabine, hydroxyurea, mercaptopurine, methotrexate, tioguanine, bleomycin, actinomycin, carboplatin, cisplatin, oxaliplatin, tretinoin, alitretinoin, bexarotene, vinblastine, vincristine, vindesine, vinorelbine, nivolumab, pembrolizumab, atezolizumab, avelumab, durvalumab, cemiplimab, pidilizumab, AMP-224, AMP-514, PDR001, BMS-936559, CK-301, imiquimod, dactolisib, gardiquimod, esiquimod or sumanirole or other Imidazoquinoline derivative, bevacizumab, thalidomide, lenalidomide, sorafenib, sunitinib, temsirolimus, axitinib, pazopanib, cabozantinib, everolimus, ramucirumab, regorafenib, vandetanib, aflibercept or ziv-aflibercept interferon alfa-n1, interferon alfa-n3, interferon beta-1b, interferon alfa-2b, interferon gamma-1b, interferon Alfa-2a, Interferon beta-1a, interferon alfacon-1, peginterferon alfa-2a, peginterferon beta-1a, salts or prodrugs or derivatives or sustained release formulations thereof or a combination thereof.

13. A compound according to any of preceding claims 1 to 5 for its use in reducing proliferation of a cell in a subject in need thereof.

14. The use of a compound according to anyone of claim 1 to 5 as dihydroorotate dehydrogenase (DHODH) inhibitor in an *in vitro* assay.
